(19)

Europäisches
Patentamt

European
Patent Office

Office européen
des brevets

(11)  **EP 2 813 183 B1**

(12)  **EUROPEAN PATENT SPECIFICATION**

(45) Date of publication and mention
of the grant of the patent:
**07.03.2018  Bulletin 2018/10**

(51) Int Cl.:
***A61B 6/00*** *(2006.01)*

(21) Application number: **14171968.2**

(22) Date of filing: **11.06.2014**

(54) **Method and apparatus for obtaining X-ray image of region of interest of target object**

Verfahren und Vorrichtung zur Erzeugung eines Röntgenbildes einer Region von Interesse eines Zielobjekts

Procédé et appareil d'obtention d'image par rayons X de région d'intérêt de l'objet cible

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2013  KR 20130066794**
**21.05.2014  KR 20140061170**

(43) Date of publication of application:
**17.12.2014  Bulletin 2014/51**

(73) Proprietor: **Samsung Electronics Co., Ltd.**
**Gyeonggi-do 443-742 (KR)**

(72) Inventors:
• **Kim, Tae-kyun**
**Seoul (KR)**

• **Kim, Sung-nam**
**Seoul (KR)**
• **Lee, Jae-chool**
**Gyeonggi-do (KR)**

(74) Representative: **D'Halleweyn, Nele Veerle Trees**
**Gertrudis et al**
**Arnold & Siedsma**
**Bezuidenhoutseweg 57**
**2594 AC The Hague (NL)**

(56) References cited:
**US-A1- 2006 198 499     US-A1- 2006 241 370**
**US-A1- 2007 071 172     US-A1- 2008 037 714**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a method and apparatus for obtaining an image including a region of interest (ROI) of a target object, and more particularly, to a method and apparatus for obtaining a medical image such as an X-ray image including a ROI of a target object by combining a first image of the target object and a second image including the whole or a portion of the ROI of the target object.

Scanning apparatuses that use X-rays are being developed and used as radiographic medical scanning apparatuses.

In a scanning apparatus using an X-ray, when an X-ray emitted from an X-ray source passes through a target, a scintillator of the scanning apparatus changes the passed X-ray to a visible ray according to the density of the target, and the visible ray is changed to an electrical signal via a photodiode of the scanning apparatus. The scanning apparatus using an X-ray generates a digital image of the target through which an X-ray has passed, by using the electrical signal. The X-ray process is required to be performed at least once to obtain an X-ray image including organs that are of interest among organs distributively located in a target object. In particular, if the X-ray process is repeatedly conducted on the entire target object a plurality of times, an amount that the target object is exposed to radiation increases, and an increase in the exposure to radiation may cause various side effects such as health problems of the target object.

Similar considerations apply for computed tomography (CT), magnetic resonance imaging (MRI) or other medical imaging systems.

In the prior art there exist various techniques for carrying out long length image, where a plurality of images are taken and combined in order to obtain a long length image. Depending on the region of interest, the length of such a long length image may vary. To that end, the movement of the source and the detector may be controlled. This movement is controlled based on external properties of the patient. Examples of such techniques are disclosed in JP 2007-260027, US 7,114,849, and US 2010/0091949.

US 2006/241370 discloses a method and system to reduce radiation exposure by panning and zooming a first image of a patient acquired by an x-ray imaging system rather than using continuous radiation fluoroscopy. The first image of a region of interest is initially acquired. Subsequently, the patient is repositioned with respect to the imaging system. Next, a second image is acquired using the imaging system and automatically resized to substantially fill the display.

US 2008/037714 discloses a CT scanner for prescanning a patient by radiating rays of a lower amount from one direction to obtain a perspective prescanning image.

US 2007/071172 discloses a device for adjusting imaging parameters for an X-ray apparatus. A user predefines on a preliminary image an image region of interest and a value of a contrast to noise ratio desired for this image region. Next, imaging parameters are calculated and the predefined region of interest is defined.

US 2006/198499 discloses a method of adapting imaging parameters for computer tomographic radiograph, wherein a three-dimensional pilot radiograph is obtained with a low dose of radiation. Next ,the region of interest is determined and an X-ray image is generated for the determined region of interest.

An object of embodiments of the invention is to provide a method and apparatus for obtaining an image of a region of interest (ROI) of a target object, wherein the exposure of the target object is limited.

According to a first aspect of the invention there is provided a method of obtaining an image of a region of interest (ROI) of a target object, according to claim 1. The method comprises obtaining a first image of the target object; providing a plurality of selectable imaging modes for reimaging the target object; and obtaining a second image of the target object according to a selected imaging mode of said plurality of imaging modes; wherein said providing of a plurality of selectable imaging modes is performed such that a selection of an imaging mode can be made after obtaining said first image and before obtaining said second image.

Embodiments of the invention are based *inter alia* on the insight that by providing a plurality of selectable imaging modes after obtaining the first image and by obtaining the second image according to a selected imaging mode, it is possible to reduce the exposure of the target object by selecting an appropriate imaging mode which is suitable for obtaining the second image so that it includes the missing portion of the ROI.

Compared to prior art techniques, the method of the invention allows selecting an appropriate imaging mode for taking a second image, based on internal properties of the patient, which are visible in the first image. This will allow determining accurately a suitable imaging mode for the second image, and hence will allow reducing the exposure of the patient.

According to an embodiment the providing of a plurality of imaging modes comprises providing a plurality of imaging modes corresponding with imaging areas having different sizes. In that way an appropriate size of the imaging area can be selected for reimaging the target object so that the ROI portion which is missing in the first image is included in the second image. Preferably, the providing of a plurality of imaging modes further comprises providing a plurality of imaging modes corresponding with different positions and/or orientations of an image source used to obtain the first and second image. More in particular, each imaging mode may define a size of an imaging area, a position and/or an orientation of an image source.

In an embodiment the obtaining of the first image and the obtaining of the second image are performed while the target object remains in a determined position. In that way the first image can be used accurately as a reference for selecting the appropriate imaging mode.

In a preferred embodiment the method further comprises: determining whether the first image includes the whole ROI; and selecting an imaging mode of said plurality of selectable imaging modes, if it is determined that the first image does not include the whole ROI; wherein the second image imaging mode is selected such that the second image includes a portion or the whole of the ROI. Note that the determining and the selecting step may be performed by the user or by the imaging apparatus. In that way, depending on the analysis of the first image, it may be determined whether or not a second image needs to be taken, and which imaging mode is most suitable for obtaining the second image.

In a preferred embodiment the determining comprises determining that a portion of the ROI is missing in the first image, and the selecting comprises selecting an imaging mode of said plurality of imaging modes corresponding with an imaging area having the smallest size capable of containing the portion which is missing in the first image.

The second image may include a portion or the whole of the ROI.

The determining whether the first image includes the ROI may include comparing a standard image of the target object and the first image.

The standard image may be selected from among previously stored images based on properties of the target object.

In an embodiment the method further comprises extracting at least one feature point and/or line related to the ROI from the first image, and the determining whether the first image includes the ROI comprises comparing the at least one feature point and/or line extracted from the first image with at least one feature point and/or line related to the ROI included in the standard image. If the number of feature points in the first image is different from the number of feature points in the standard image, it may be determined that the first image does not include the whole ROI, and/or, if a portion of the at least one line present in the standard image is missing in the first image, it may be determined that the first image does not include the whole ROI. In a possible embodiment the comparing of the at least one feature point and/or line extracted from the first image with the feature point and/or line related to the ROI included in the standard image comprises comparing the number and/or a position of the at least one feature point and/or line related to the ROI included in the standard image and the number and/or a position of the at least one feature point and/or line extracted from the first image. Using feature points and/or lines allows for a fast and effective comparing of the first image with a standard image, and results in a reliable and accurate determining whether the first image includes the whole ROI.

The method may further include extracting at least one feature point related to the ROI from the first image. The determining whether the first image includes the ROI may include comparing the at least one feature point extracted from the first image with at least one feature point related to the ROI included in the standard image.

The number and a position of the at least one feature point may be differently defined in advance according to a size and a position of the ROI.

The comparing of the at least one feature point extracted from the first image with the at least one feature point related to the ROI included in the standard image may include comparing the number and a position of the at least one feature point related to the ROI included in the standard image and the number and a position of the at least one feature point extracted from the first image.

The method may further include extracting a boundary line of the ROI from the first image. The determining whether the first image includes the ROI may include comparing the boundary line extracted from the first image and a boundary line of the ROI included in the standard image.

The comparing of the boundary line extracted from the first image and the boundary line of the ROI included in the standard image may include determining a similarity between the boundary line extracted from the first image and the boundary line of the ROI included in the standard image.

The imaging mode may include a whole imaging mode in which the entire ROI of the target object is reimaged and a partial imaging mode in which a portion of the ROI of the target object is reimaged.

The method may further include determining a portion of the ROI to be additionally imaged, when the partial imaging mode is selected. The second image including the determined portion may be obtained.

The determining of the portion to be additionally imaged may include: estimating a portion of the ROI that is not included in the first image; and determining an additional imaging area including the estimated portion by using size information of the first image.

The estimating of the portion of the ROI not included in the first image may include determining a size and a position of the portion not included in the first image based on the size and the position of the ROI included in the standard image.

The size information of the first image may include at least one of height information and width information of the first image.

The determining of the additional imaging area may include determining an imaging area such that all portions of the ROI corresponding to the determined size and position of the portion not included in the first image are included in the additional imaging area based on at least one of the height information and the width information of the first image.

The method may further include receiving an additional imaging area of the target object, as an external input signal, when the partial imaging mode is selected. The second image corresponding to the received additional imaging area may be obtained.

The method may further include setting an imaging condition based on the selected imaging mode. The obtaining of the second image may include obtaining an X-ray image of the target object according to the set imaging condition.

The imaging condition may include at least one of a position of an imaging source, an amount of collimation, a position of an image detector, and an image resolution. The imaging source may be e.g. an X-ray source and the image detector may be e.g. an X-ray detector.

The method may further include combining the first image and the second image.

The second image may include at least one template to be used in combining the second image with the first image.

The combining of the first image and the second image may include stitching the second image with the first image by using the at least one template.

The at least one template may be differently set based on at least one of properties of the target object, a size of the ROI, and a position of the ROI.

The second image may include a plurality of templates to be used in combining the second image with the first image, wherein the combining of the first image and the second image includes: applying different weights to the plurality of templates; and stitching the obtained first image to the second image based on the applied weights.

According to a second aspect of the present invention, there is provided a method of obtaining an image of a region of interest (ROI) of a target object, the method including: obtaining a first image of the target object; determining whether the first image includes the ROI; selecting an imaging mode to image the target object based on the determining; and obtaining a second image of the target object according to the selected imaging mode.

The preferred features described above for the first aspect of the invention, may also be included in the method according to the second aspect of the invention.

According to a third aspect of the invention there is provided an apparatus for obtaining an image of a region of interest (ROI) of a target object, according to claim 12. The apparatus comprises an image obtaining unit configured for obtaining a first image of the target object; an image mode providing unit configured for providing a plurality of selectable imaging modes for reimaging the target object, after having obtained the first image; wherein the imaging obtaining unit is further configured for obtaining a second image according to a selected imaging mode of said plurality of imaging modes.

In an embodiment the image mode providing unit is configured for providing a plurality of imaging modes corresponding with imaging areas having different sizes.

In an embodiment the image mode providing unit is configured providing a plurality of imaging modes corresponding with different positions and/or orientations of an image source used to obtain the first and second image.

In an embodiment the apparatus further comprises an area determining unit configured for determining whether the first image includes the whole ROI; and an imaging mode selecting unit configured for selecting an imaging mode of said plurality of imaging modes to reimage the target object if it is determined that the first image does not include the whole ROI. Preferably, the area determining unit is configured for determining which portion of the ROI is missing in the first image, and the image mode selecting unit is configured for selecting an imaging mode of said plurality of imaging modes corresponding with an imaging area having the smallest size capable of containing the portion which is missing in the first image.

In an embodiment the area determining unit is configured to compare a standard image of the target object and the first image; wherein preferably the standard image is selected based on properties of the target object from among previously stored images.

In an embodiment the apparatus further comprises an extracting unit configured for extracting at least one feature point and/or line related to the ROI from the first image, and the determining unit is configured for determining whether the first image includes the ROI by comparing the at least one feature point and/or line extracted from the first image with at least one feature point and/or line related to the ROI included in the standard image. If the number of feature points in the first image is different from the number of feature points in the standard image, it may be determined that the first image does not include the whole ROI, and/or, if a portion of the at least one line present in the standard image is missing in the first image, it may be determined that the first image does not include the ROI. In a possible embodiment the comparing of the at least one feature point and/or line extracted from the first image with the feature point and/or line related to the ROI included in the standard image comprises comparing the number and/or a position of the at least one feature point and/or line related to the ROI included in the standard image and the number and/or a position of the at least one feature point and/or line extracted from the first image.

The apparatus may further include a feature point extracting unit for extracting at least one feature point related to the ROI from the first image, wherein the determining unit further includes a comparing unit that compares the at least one feature point detected from the first image with at least one feature point of the ROI included in the standard image.

The number and a position of the at least one feature point may be defined differently in advance according to a size and a position of the ROI.

The comparing unit may compare the number and a position of a feature point of the ROI included in the standard image and the number and a position of the at least one feature point extracted from the first image.

The apparatus may further include a boundary line extracting unit for extracting a boundary line of the ROI from the first image, wherein the determining unit further includes a comparing unit that compares the boundary line extracted from the first image with a boundary line of the ROI included in the standard image.

The comparing unit may determine a similarity between the boundary line extracted from the first image and a boundary line of the ROI included in the standard image.

The imaging mode may include a whole imaging mode in which the entire ROI of the target object is reimaged and a partial imaging mode in which a portion of the ROI of the target object is reimaged.

The apparatus may further include an additional imaging determining unit that determines a portion of a ROI that is to be additionally imaged, as a partial imaging mode is selected.

The additional imaging determining unit may include an insufficient portion estimating unit that estimates a portion of a ROI not included in the first image and an additional imaging area determining unit that determines an additional imaging area including the portion estimated by using the insufficient portion estimating unit by using size information of the first image.

The insufficient portion estimating unit may determine a size and a position of a portion not included in the first image based on a size and a position of a ROI included in a standard image.

The size information of the first image may include at least one of height information and width information of the first image.

The additional imaging area determining unit may determine an imaging area such that all of portions of a ROI corresponding to the size and the position of the portion determined by using the insufficient portion estimating unit are included in the additional area, based on at least one of the height information and the width information of the first image.

The apparatus may further include an external input receiving unit that receives an additional imaging area of a target object, as an external input signal, as a partial imaging mode is selected, and a second image corresponding to the received additional imaging area received may be obtained.

The apparatus may further include an imaging condition setting unit that sets an imaging condition based on the selected imaging mode, and a second image may be obtained as an image of a target object according to a set imaging condition.

An imaging condition may include at least one of a position of an imaging source (e.g. an X-ray source), an amount of collimation, a position of an imaging detector (e.g. an X-ray detector), and an image resolution.

The apparatus may further include an image combining unit that combines an obtained first image and an obtained second image.

The second image may include at least one template to be used in combining the second image with the first image, and the image combining unit may stitch an obtained first image and an obtained second image by using at least one template.

The at least one template may be set differently based on at least one of properties of a target object, a size of a ROI, and a position of a ROI.

The second image may include a plurality of templates to be used in combining the second image with the first image, and the image combining unit may further include a weight applying unit that applies different weights to a plurality of templates. Also, the image combining unit may stitch an obtained first image and an obtained second image based on the applied weights.

According to a fourth aspect of the present invention, there is provided an apparatus for obtaining an X-ray image of a region of interest (ROI) of a target object, the apparatus including: an image obtaining unit for obtaining a first image of the target object; a determining unit for determining whether the first image includes the ROI; and an imaging mode selecting unit for selecting an imaging mode to reimage the target object based on the determining, wherein a second image of the target object is obtained by using the image obtaining unit according to the selected imaging mode, and the second image includes the whole or a portion of the ROI.

The preferred features described above for the third aspect of the invention, may also be included in the apparatus according to the fourth aspect of the invention.

According to a fifth aspect of the present invention, there is provided a method of obtaining an image of a region of interest (ROI) of a target object, the method including: obtaining a first image of the target object; obtaining a second image of the target object; and generating an image including the ROI of the target object by using the first image and the second image, wherein the second image has a different size from the first image and includes a portion or the whole of the ROI.

The second image may be smaller than the first image.

According to a sixth aspect of the present invention, there is provided a method of obtaining an image of a region of interest (ROI), the method comprising: determining sizes of a first image and a second image that are to be imaged based on a size of the ROI; obtaining the first image of the target object based on the determined size; obtaining the second image of the target object based on the determined size; and generating an image including the ROI of the target object by using the first image and the second image, wherein the second image has a different size from the first image and includes a portion or the whole of the ROI.

The generating of an image including the ROI of the target object by using the first image and the second image may include generating an image by combining the first image and the second image by overlapping the first image and the second image by a predetermined size.

According to a seventh aspect of the present invention, there is provided an apparatus for obtaining an image of a region of interest (ROI) of a target object, comprising: an image obtaining unit configured for obtaining a first image and a second image of the target object; and an image generating unit configured for generating an image including the ROI of the target object by using the first image and the second image, wherein the second image has a different size from the first image and includes a portion or the whole of the ROI.

The second image may be smaller than the first image.

According to a seventh aspect of the present invention, there is provided an apparatus for obtaining an image of a region of interest (ROI) of a target object, comprising: an image size determining unit configured for determining sizes of a first image and a second image to be imaged based on a size of the ROI; an image obtaining unit configured for obtaining the first image and the second image of the target object based on the determined sizes; and an image generating unit configured for generating an image including the ROI of the target object by using the first image and the second image, wherein the second image has a different size from the first image and includes a portion or the whole of the ROI.

The image generating unit may be configured to generate an image by combining the first image and the second image by overlapping the first image and the second image by a predetermined size.

The predetermined size by which the first image and the second image may be overlapped is in the range from about 35 mm to about 90 mm.

According to another aspect of the present invention, there is provided a computer readable recording medium having embodied thereon a program for executing any one of the steps of any one of the embodiments of the method described above.

## BRIEF DESCRIPTION OF THE DRAWINGS

The above and other features and advantages of the present invention will become more apparent by describing in detail exemplary embodiments thereof with reference to the attached drawings in which:

FIG. 1 illustrates an exemplary image including a kidney, a ureter, and a bladder of a target object;

FIGS. 2A through 2C are schematic views illustrating a method of obtaining an X-ray image including a ROI of a target object, according to an embodiment of the present invention;

FIGS. 3A and 3B are flowcharts illustrating a method of obtaining an X-ray image including a ROI of a target object, according to an embodiment of the present invention;

FIG. 4 illustrates an operation of determining whether a first image includes a ROI, according to an embodiment of the present invention;

FIGS. 5A through 5E illustrate standard images according to an embodiment of the present invention;

FIG. 6 is a flowchart illustrating a method of determining whether a first image includes a ROI, according to another embodiment of the present invention;

FIGS. 7A through 7E illustrate feature points according to an embodiment of the present invention;

FIG. 8 is a flowchart illustrating a method of determining whether a first image includes a ROI, according to another embodiment of the present invention;

FIGS. 9A through 9E illustrate boundary lines according to an embodiment of the present invention;

FIGS. 10A and 10B illustrate a method of selecting a imaging mode according to an embodiment of the present invention;

FIG. 11 is a flowchart illustrating a method of determining an additional imaging area when a partial imaging mode is selected, according to an embodiment of the present invention;

FIGS. 12A and 12B illustrate an estimated example of a portion to be additionally imaged, according to an embodiment of the present invention;

FIGS. 13A and 13B illustrate an estimated example of a portion to be additionally imaged, according to another embodiment of the present invention;

FIG. 14 is a flowchart illustrating a method of determining a portion to be additionally imaged, when a partial imaging mode is selected, according to another embodiment of the present invention;

FIGS. 15A through 15C illustrate an example, in which a portion to be additionally imaged is received as an external input signal, according to an embodiment of the present invention;

FIG. 16 illustrates an example of selecting a imaging condition according to an embodiment of the present invention;

FIG. 17 illustrates an example of selecting a imaging condition according to another embodiment of the present invention;

FIG. 18 is a flowchart illustrating a method of combining a first image and a second image, according to an embodiment of the present invention;

FIG. 19 illustrates an example of combining a first image and a second image, according to an embodiment of the present invention;

FIGS. 20A and 20B illustrate an example of combining a first image and a second image, according to another embodiment of the present invention;

FIGS. 21A and 21B illustrate an example of combining a first image and a second image, according to another embodiment of the present invention;

FIG. 22 is a block diagram illustrating an apparatus for obtaining an X-ray image including a ROI of a target object, according to an embodiment of the present invention;

FIG. 23 is a block diagram illustrating an apparatus for obtaining an X-ray image including a ROI of a target object, according to another embodiment of the present invention;

FIG. 24 is a block diagram illustrating an apparatus for obtaining an X-ray image including a ROI of a target object, according to another embodiment of the present invention;

FIG. 25 is a block diagram illustrating an X-ray image obtaining apparatus that further includes an additional imaging determining unit, according to an embodiment of the present invention;

FIG. 26 is a block diagram illustrating an X-ray image obtaining apparatus that further includes an external input receiving unit and an additional imaging determining unit, according to an embodiment of the present invention;

FIG. 27 is a block diagram illustrating an X-ray image obtaining apparatus that further includes a imaging condition setting unit, according to an embodiment of the present invention;

FIG. 28 is a block diagram illustrating an X-ray image obtaining apparatus that further includes an image combining unit, according to an embodiment of the present invention;

FIG. 29 is a block diagram illustrating an X-ray image obtaining apparatus that further includes an image combining unit, according to another embodiment of the present invention; and

FIG. 30 is a block diagram illustrating an apparatus for obtaining an X-ray image including a ROI of a target object, according to another embodiment of the present invention.

FIG. 31 illustrates an X-ray tube and a detector that are used to obtain an image of a target object by sensing an X-ray transmitting through the target object; and

FIG. 32 shows individual images respectively corresponding to imaging areas of a target object, according to an embodiment of the present invention.

## DETAILED DESCRIPTION OF THE INVENTION

Terminology used herein will now be briefly described, and the present invention will be described in detail.

Although general terms widely used at present were selected as terminology used in the present invention while considering the functions of the present invention, they may vary according to intentions of one of ordinary skill in the art, judicial precedents, the advent of new technologies, and the like. Terms arbitrarily selected by the applicant of the present invention may also be used in a specific case. In this case, their meanings need to be given in the detailed description of the present invention. Hence, the terms must be defined based on the meanings of the terms and the contents of the entire specification, not by simply stating the terms themselves.

It will be understood that the terms "comprises" and/or "comprising" or "includes" and/or "including" when used in this specification, specify the presence of stated elements, but do not preclude the presence or addition of one or more other elements. Terms such as "... unit" and "module" stated in the specification denote units that process at least one function or operation, and they may be implemented by using hardware, software, or a combination of hardware and software.

Expressions such as "at least one of," when preceding a list of elements, modify the entire list of elements and do not modify the individual elements of the list.

In the entire specification, an "image" may refer to multi-dimensional data representing discrete image elements (e.g., pixels of a two-dimensional (2D) image and voxels of a three-dimensional (3D) image). For example, an image may include a medical image of a target object, which is obtained using X-rays, computed tomography (CT), magnetic resonance imaging (MRI), ultrasonography, or other medical imaging systems. In the exemplary embodiments described below the medical image is obtained using X-rays, but the skilled person understands that the principles of the invention are equally applicable when the image is obtained in a different way.

Also, a "target object" in the specification may refer to a human being or an animal or a part of a human being or an animal. For example, the target object may include a liver, heart, uterus, brain, breast, abdomen, or blood vessels. In addition, the "target object" may include a phantom. A phantom may refer to a material that has a highly approximate volume to a density of an organism and an effective atom number and may include a spherical phantom that has similar properties to those of a human body.

In the entire specification, a "user" may be a medical expert, such as a doctor, a nurse, a medical technologist, or a medical image expert, but the present invention is not limited thereto.

The present invention will now be described more fully with reference to the accompanying drawings, in which exemplary embodiments of the invention are shown. The invention may, however, be embodied in many different forms and should not be construed as being limited to the embodiments set forth herein. In the drawings, parts irrelevant to the description are omitted for simplicity of explanation, and like numbers refer to like elements throughout.

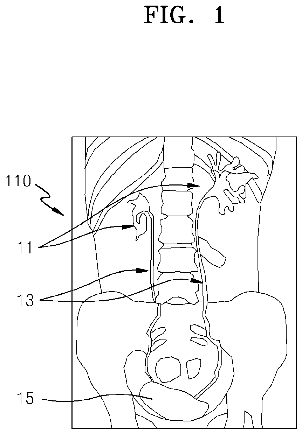

FIGS. 1A and 1B are schematic views illustrating a method of obtaining an X-ray image including a region of interest (ROI) of a target object, according to the conventional art.

According to the conventional art, when an organ that is of interest to a user is not included in an X-ray image of a target object, at least one of an X-ray source unit and an X-ray detecting unit are moved to reimage the whole target object so that all organs of interest are included in an X-ray image.

For example, when an X-ray image 110 of a target object is obtained, and the whole or a portion of a tissue that is of interest to the user (e.g., a lung, kidney, or bladder) is not included in the obtained X-ray image, an image of an area 120 including the tissue of interest to the user has to be acquired. In this case, a position of the target object or a imaging device may be adjusted such that the tissue of interest to the user is completely included in an X-ray image, thereby obtaining an X-ray image 130 of the target object again.

For example, to obtain the X-ray image 130 including both a lung and a bladder of a target object, reimaging may be conducted at least twice according to the skills of the user regarding an X-ray imaging apparatus. In other words, as internal structures may vary according to target objects, even a skilled user may have to repeat X-ray imaging of a target object to obtain an X-ray image that contains all areas of interest to the user. For example, positions of organs may be different according to the gender, age, or body type of a target object, and thus, it is difficult to always capture an image that contains every area of interest to the user (e.g., tissues such as a lung or a bladder).

The more times an X-ray image of a target object is recaptured, the more a cumulative amount of X-ray irradiated to the target object increases. In other words, the higher the number of times X-ray reimaging of the target object is performed, the greater the amount of exposure of the target object to radiation.

In addition, when a position of a target object or an X-ray imaging apparatus has to be adjusted so that all areas of interest to the user are included in an X-ray image, the time taken for X-ray imaging also increases by a period of time needed for the adjustment of the position thereof.

Accordingly, if not all areas of interest to the user are included in the obtained X-ray image, X-ray imaging a smallest area including portions (e.g., insufficient portions) that were missing or not contained in the previously obtained X-ray image may be performed instead of X-ray reimaging the whole of the target object, and the captured plurality of images may be combined, thereby reducing unnecessary exposure of the target object to radiation and reducing a imaging time.

FIG. 1 illustrates an exemplary image including a kidney, a ureter, and a bladder of a target object.
According to the conventional art, when an organ that is of interest to a user is not included in an X-ray image of a target object, at least one selected from an X-ray source unit and an X-ray detecting unit is moved to reimage the whole target object so that all organs of interest are included in an X-ray image.

For example, when an X-ray image 110 of a target object is obtained, and the whole or a portion of a tissue that is of interest to the user (e.g., a kidney 11, a ureter 13, and a bladder 15) is not included in the obtained X-ray image, an image of an area including the tissue of interest to the user has to be acquired. In this case, a position of the target object or a imaging device may be adjusted such that the tissue of interest to the user (e.g., the kidney 11, the ureter 13, and the bladder 15) is completely included in an X-ray image, thereby obtaining the X-ray image of the target object again.

For example, to obtain an X-ray image including all of the kidney 11, the ureter 13, and the bladder 15 of a target object, reimaging may be conducted at least twice according to skills of the user regarding an X-ray imaging apparatus. In other words, as internal structures may vary according to target objects, even a skilled user may have to repeat X-ray imaging of a target object to obtain an X-ray image that contains all areas of the interest to the user. For example, positions of organs may be different according to the gender, age, or body type of a target object, and thus, it is difficult to always capture an image that contains every area of interest to the user (e.g., tissues such as the kidney 11, the ureter 13, and the bladder 15).

The more times an X-ray image of a target object is recaptured, the more a cumulative amount of X-ray irradiated to the target object increases. In other words, the higher the number of times X-ray reimaging of the target object is performed, the greater the amount of exposure of the target object to radiation.

In addition, when a position of a target object or an X-ray imaging apparatus has to be adjusted so that all areas of interest to the user are included in an X-ray image, the time taken for X-ray imaging also increases by a period of time needed for the adjustment of the position thereof.

Accordingly, if not all areas of interest to the user are included in the obtained X-ray image, X-ray imaging of a smallest area including portions (e.g., insufficient portions) that were missing or not contained in the previously obtained X-ray image may be performed instead of X-ray reimaging the whole of the target object, and the captured plurality of images may be combined, thereby reducing unnecessary exposure of the target object to radiation and reducing a imaging time.

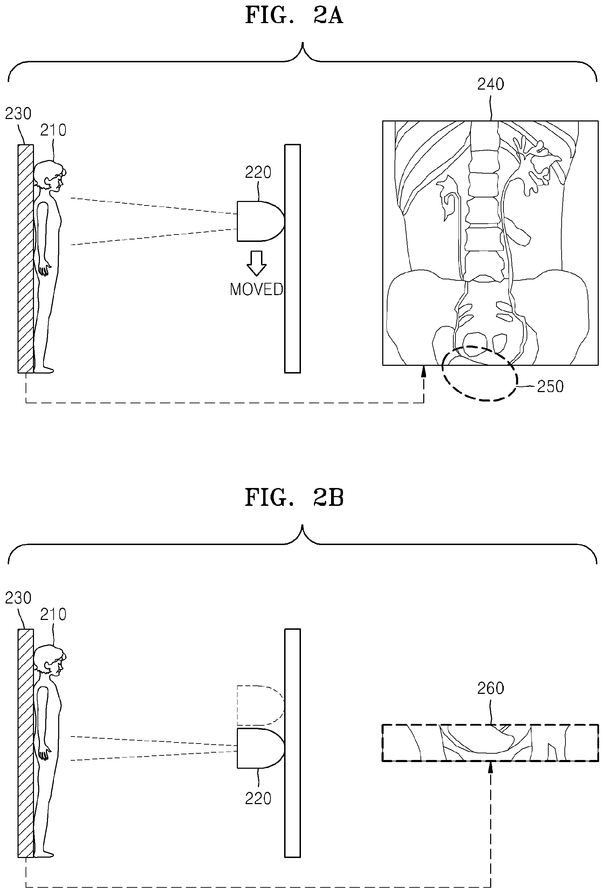

FIGS. 2A through 2C are schematic views illustrating a method of obtaining an X-ray image including a ROI of a target object according to an embodiment of the present invention.

As illustrated in FIG. 2A, an X-ray image 240 of a target object 210 located between an X-ray source 220 and an X-ray detector 230 may be obtained. For example, a user may have intended to obtain an X-ray image that includes both a lung and a bladder of the target object 210 but the X-ray image 240 obtained may not include a bladder 250.

In order to obtain an image 260 of the bladder 250 which is not included by previous imaging, as illustrated in FIG. 2B, imaging may be conducted after adjusting a position of the X-ray source 220 or a position of the target object 210. Also, a size and a position of a collimator that adjusts a range of X-ray radiation, i.e. that adjust the imaging area may be adjusted. To that end there are provided a plurality of selectable imaging modes from which a user or the imaging apparatus may select a suitable imaging mode for imaging the missing portion. This selection may be based on a determination, by the user or by the imaging apparatus, of the missing portion in the first image, and in particular of the size and location of the missing portion. By providing imaging modes that correspond with imaging areas having different sizes and/or different shapes, a suitable size and/or shape may be selected. When a particular imaging mode is selected, the size of the imaging area may be set by adjusting a collimator of the imaging source and/or by placing a cover with a suitable sized opening between the imaging source and the target object.

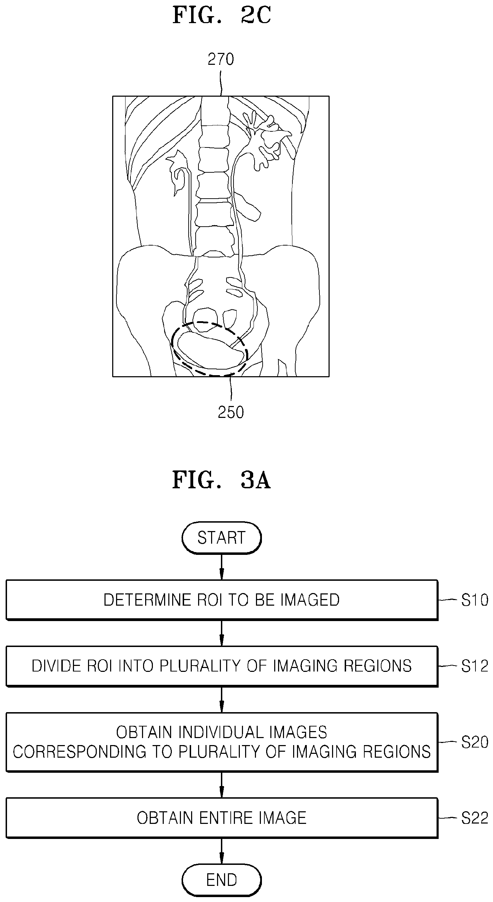

As illustrated in FIG. 2C, by combining a plurality of X-ray images, (e.g., the X-ray images 240 and 260), an X-ray image 270 including all ROIs may be obtained.

That is, according to the current embodiment of the present invention, in order to obtain an image of the bladder 250, the image 260 may be obtained by irradiating an X-ray to a minimal extent such that the bladder 250 is included in the image 260, and organs such as a lung which is disposed above the bladder 250 and is already imaged does not have to be exposed to radiation again. In the illustrated example the different imaging modes may e.g. correspond with rectangular imaging areas having a different height and/or a different width, and there may be selected an imaging mode having a height corresponding with the height of the missing portion as derived from the first image either automatically by the imaging apparatus or by a user. In the latter case, there may be provided an input interface where the user can select a suitable imaging mode, e.g. by specifying the height of the missing portion.

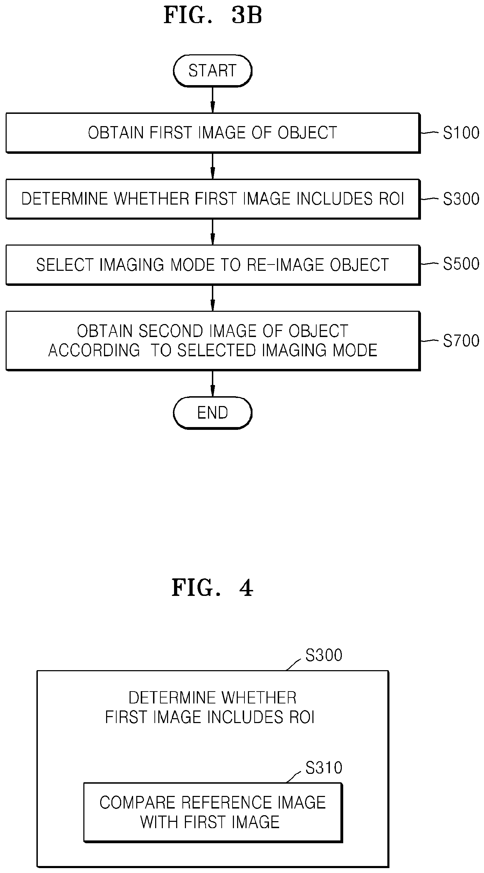

FIGS. 3A and 3B are flowcharts illustrating a method of obtaining an X-ray image including a ROI of a target object, according to an embodiment of the present invention. FIG. 3A is a flowchart of a main imaging operation. FIG. 3B is a flowchart of a correction imaging operation or a supplementary imaging operation that is in connection with the main imaging operation in regard to FIG. 2.

In operation S10, an ROI to be imaged may be determined. The determined ROI may be divided into a plurality of imaging regions in operation S12.

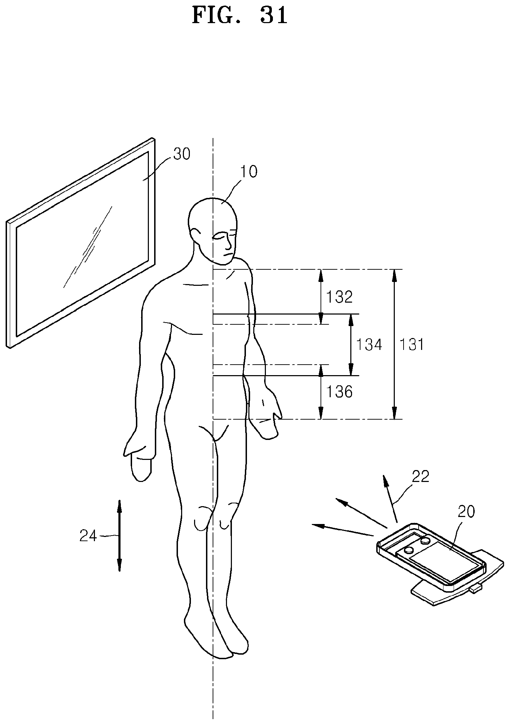

FIG. 31 illustrates an X-ray tube 20 and a detector 30 that are used to obtain an image of a target object by sensing an X-ray 22 transmitting through a target object 10.

A size of an X-ray tube or a detector of a medical equipment including an X-ray imaging equipment or a resolution of an image obtained by using the equipment may be limited so that an image including a broad ROI with respect to a target object may not be obtained through one-time imaging. In other words, an image including a broad ROI with respect to a target object may be obtained as a combination image obtained by combining small image portions that are imaged with respect to the target object. An image of a portion of a target object that may be used to obtain a combination image with respect to the target object may be obtained by rotating at least one selected from the X-ray tube 20 and the detector 30 or by, for example, vertically moving the X-ray tube 20 or the detector 30 in a perpendicular direction 24. A combination image with respect to the target object 10 may be obtained by combining images of portions of the target object described above by using various image combination methods. For example, a ROI 131 of a target object may be divided into imaging regions 132, 134, and 136 of the same or different lengths in a preset direction that is parallel to the perpendicular direction 24. An individual image of each imaging region may be obtained.

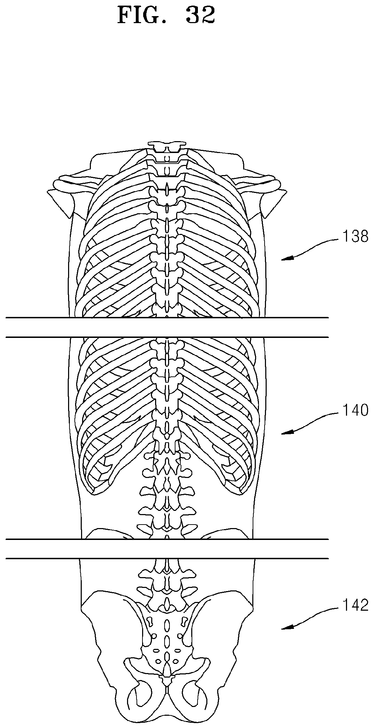

FIG. 32 shows individual images respectively corresponding to imaging areas of a target object, according to an embodiment of the present invention.

Referring to FIGS. 3A and 32, for example, individual images 138, 140, and 142 respectively corresponding to the imaging regions 132, 134, and 136 may be obtained in operation S20. The individual images 138, 140, and 142, for example, may be combined to a whole X-ray image in which a ROI of a target object is included.

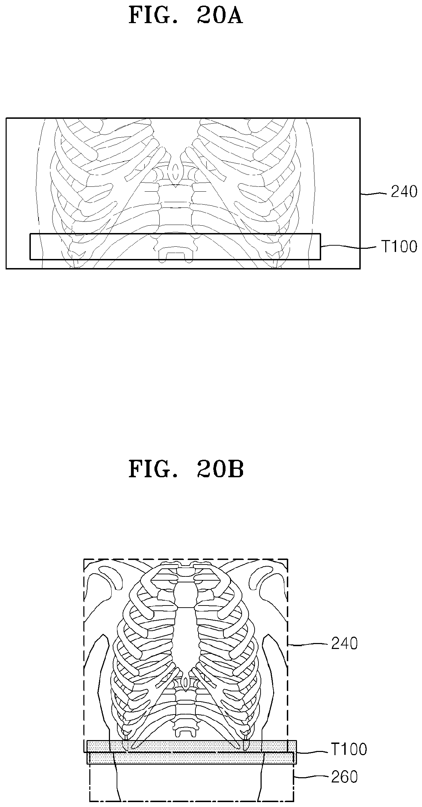

Operation S10, S12, S20 or S22 described above may be performed by using an apparatus 2000 described below with reference to FIG. 20. For example, operation S10, S12, S20 or S22 may be performed by using the imaging obtaining unit 2100 of the apparatus 2000. Also, operation S10, S12, S20 or S22 may be performed by using another processor or another apparatus of the apparatus 2000. In addition, operations S12 and S20 may be omitted.

As illustrated in FIG. 3B, the method of obtaining an X-ray image including a ROI of a target object, according to the current embodiment of the present invention, may include: obtaining a first image of a target object (S100); determining whether the first image includes a ROI (S300); selecting a imaging mode to reimage the target object based on a result of the determining (S500); and obtaining a second image of the target object according to the selected imaging mode (S700). The first image obtained in operation S22 of FIG. 3A may correspond to an X-ray image in which a ROI of a target object is included.

The second image according to the current embodiment of the present invention may include the whole or a portion of the ROI. For example, the first image may be analyzed in order to reduce a degree of exposure of the target object to radiation, and a imaging mode may be selected, in which a second image including a portion lacking in the first image is imaged. The above operations may be performed on a target object existing at a predetermined position, and the first image of the target object existing at the predetermined position may be a standard for selecting a imaging mode.

Also, a imaging mode may be selected from among a plurality of imaging modes corresponding to imaging areas having different sizes. The plurality of imaging modes may include a whole imaging mode and a partial imaging mode.

FIG. 4 illustrates an operation of determining whether the obtained first image includes a ROI, according to an embodiment of the present invention.

The determining whether the first image includes a ROI (S300) may include comparing a standard image of the target image and the first image (S310). The comparing images according to the current embodiment of the present invention may include a series or operations that are conducted to determine a similarity between a plurality of images, which will be described later.

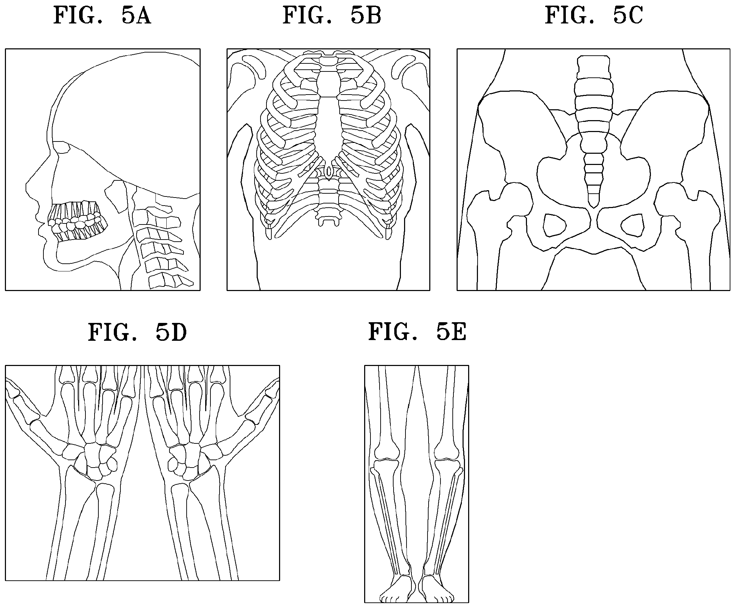

FIGS. 5A through 5E illustrate standard images according to an embodiment of the present invention.

FIG. 5A illustrates an example of a standard image of a skull and cervical vertebrae of a target object. FIG. 5B is an example of a standard image of a chest of the target object. FIG. 5C is an example of a standard image of a lower abdominal portion of the target object. FIG. 5D is an example of a standard image of arms of the target object. FIG. 5E is an example of a standard image of legs of the target object.

The standard images of the target object may be statistically determined for respective portions (for example, body parts) of the target object and stored in a database in advance. For example, predetermined elements (e.g., the number of ribs or positions of diaphragms included in the images) found in X-ray images of a chest of a plurality of target objects are extracted, and the most frequently extracted elements are defined as common features based on a frequency that the elements appear in X-ray images of the chest of the plurality of target object, and the X-ray images of the chest of the target objects including the common features may be defined as the standard images and stored.

In addition, the standard images according to the current embodiment of the present invention may be selected based on properties of a target object that is to be imaged, from among images of the plurality of target objects stored in advance. The properties of the target object according to an embodiment of the present invention may include the age, gender, height, weight, body type, part to be imaged, medical history of the target object to be imaged and so on.

In other words, a standard image may be selected based on at least one of the age, gender, height, weight, body type, part to be imaged, and medical history of the target object to be imaged, from among images of a plurality of target objects that are stored in advance. For example, from among the images of a plurality of target objects that are stored in advance, a standard image of a chest of a child and that of a chest of an adult may be differently selected. Also, as illustrated in FIGS. 5A through 5E, standard images may be differently selected according to a portion of a target object to be imaged.

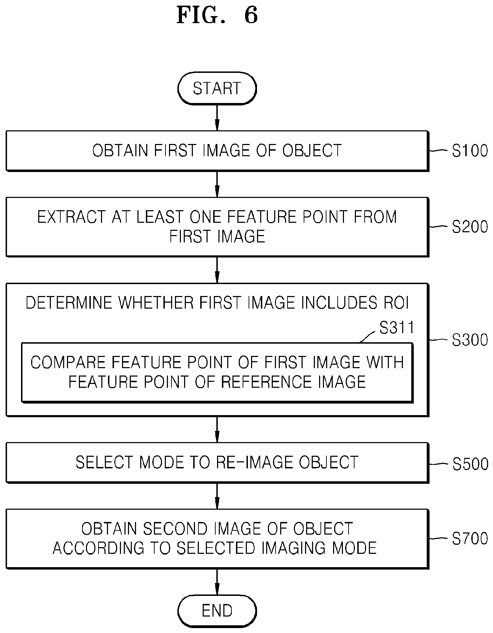

FIG. 6 is a flowchart illustrating a method of determining whether a first image includes a ROI, according to another embodiment of the present invention.

The method of determining whether a first image includes a ROI according to the current embodiment of the present invention may further include extracting at least one feature point related to a ROI from the first image (S200). Determining whether the first image includes a ROI (S300) may include comparing a feature point extracted from the first image with a feature point regarding a ROI included in a standard image (S311).

For example, whether the first image includes a ROI may be determined by using a scale-invariant feature transform (SIFT) method using a feature point of a target object or a model-based matching method.

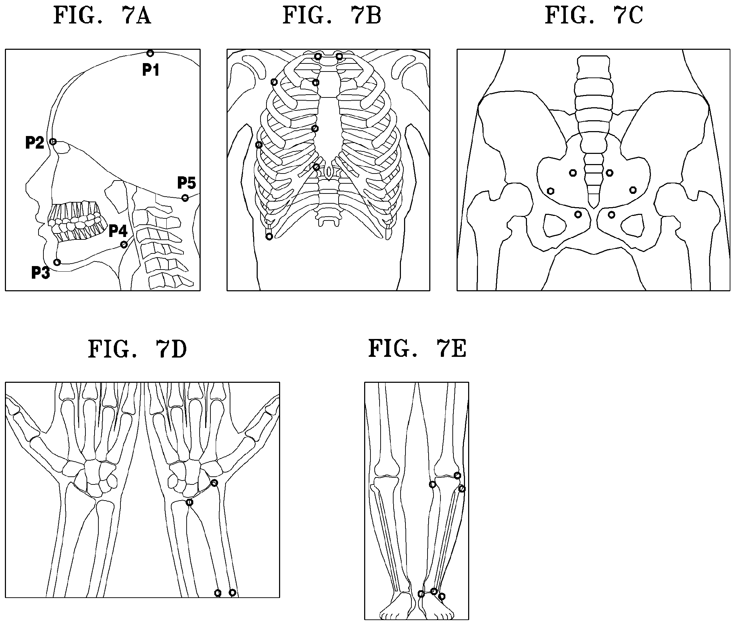

FIGS. 7A through 7E illustrate feature points according to an embodiment of the present invention.

The number and a position of at least one feature point according to an embodiment of the present invention may be defined in advance according to a size and a position of a ROI.

The feature point according to an embodiment of the present invention may include a predetermined point to distinguish, for example, tissues included in an X-ray image of a target object. For example, the feature point may be used as an identifier to identify at least one of a shape, size, and position of a tissue or the like included in an X-ray image of a target object.

As illustrated in FIG. 7A, for example, a plurality of feature points (e.g., feature points P1 through P5) may indicate that a imaged tissue is a skull of a target object. That is, the skull of the target object may be identified in an X-ray image based on a plurality of feature points (e.g., the feature points P1 through P5).

As illustrated in FIGS. 7B through 7E, at least one feature point of each portion of a target object may be determined in advance.

An operation of comparing a feature point extracted from a first image and a feature point of a ROI included in a standard image, according to an embodiment of the present invention, may include comparing the number and a position of a feature point of the ROI included in the standard image and the number and a position of a feature point extracted from the first image.

For example, when a skull of a target object is a ROI, and only some feature points (e.g., P1', P2', and P5') are found in the first image that is obtained in order to capture an image of the skull of the target object, feature points (e.g., P3' and P4') of the first images corresponding to the feature points P3 and P4 from among a plurality of feature points (e.g., P1 through P5) included in the standard image do not exist. Thus, it may be determined that the first image does not include a ROI. That is, a lower jaw corresponding to the feature points P3 and P4 of the standard image may be determined as not being included in the first image.

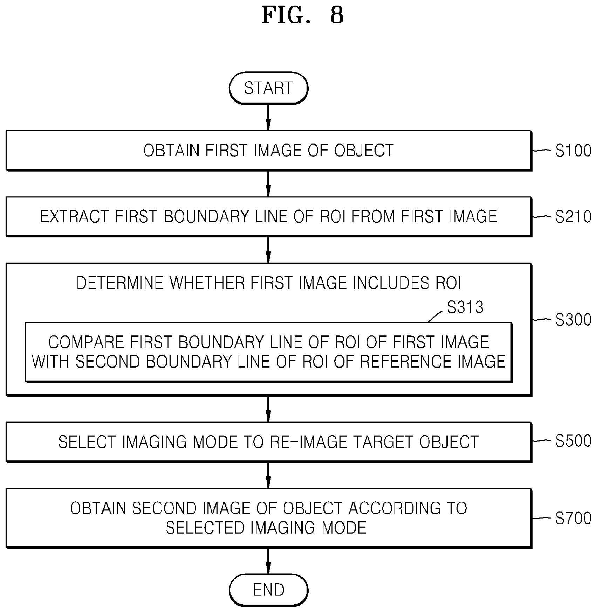

FIG. 8 is a flowchart illustrating a method of determining whether a first image includes a ROI, according to another embodiment of the present invention.

The method of determining whether a first image includes a ROI, according to the current embodiment of the present invention, may further include extracting a boundary line of the ROI from the first image (S210).

Determining whether the first image includes the ROI (S300) may include comparing a boundary line extracted from the first image and a boundary line of the ROI included in a standard image (S313).

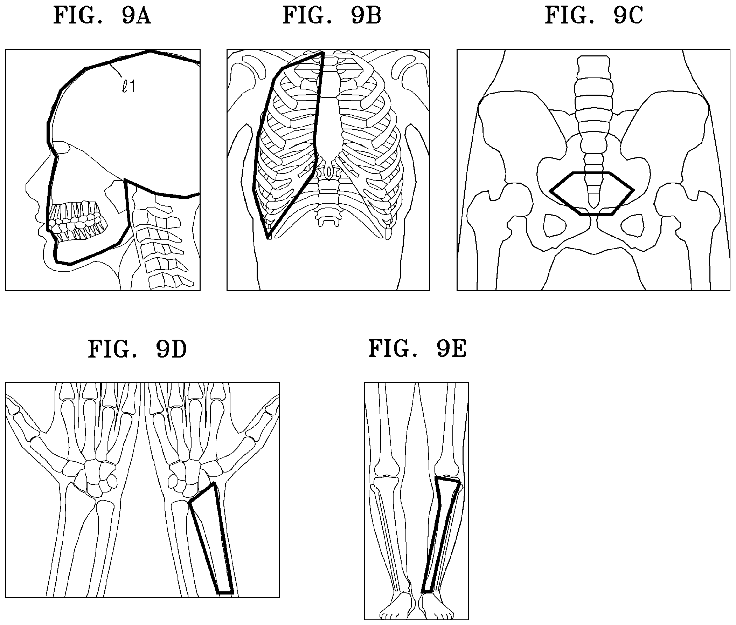

FIGS. 9A through 9E illustrate boundary lines according to an embodiment of the present invention.

Similarly to the feature points, the boundary line according to the current embodiment of the present invention may include a predetermined line used to distinguish, for example, a tissue included in an X-ray image of a target object. The line may be a solid line, a dotted line, or an alternating long and short dashed line, but is not limited thereto. The boundary line may be used as an identifier to identify at least one of a shape, size, and position of the tissue included in the X-ray image of the target object.

As illustrated in FIG. 9A, for example, a skull of a target object may be identified by a boundary line l1. Also, as illustrated in FIGS. 9B through 9E, a boundary line of each portion of the target object may be edited and may be determined in advance.

Comparing a boundary line extracted from a first image and a boundary line of a ROI included in a standard image, according to the current embodiment of the present invention, may include determining a similarity between the boundary line extracted from the first image and the boundary line of the ROI included in the standard image.

For example, by determining a similarity between two boundary lines l1 and l1' based on whether a boundary line (e.g., l1') extracted from the first image is cut or deformed compared to a boundary line (e.g., l1) of the ROI included in the standard image, it may be determined whether the first image includes the ROI or not.

For example, when a similarity between the boundary line 11 of the ROI included in the standard image and the boundary line l1' extracted from the first image is about 95% or greater, it may be determined that the ROI is included in the first image, and when the similarity is less than about 75%, it may be determined that the ROI is not included in the first image. However, a value of the similarity used as a reference for the above determination is not limited thereto.

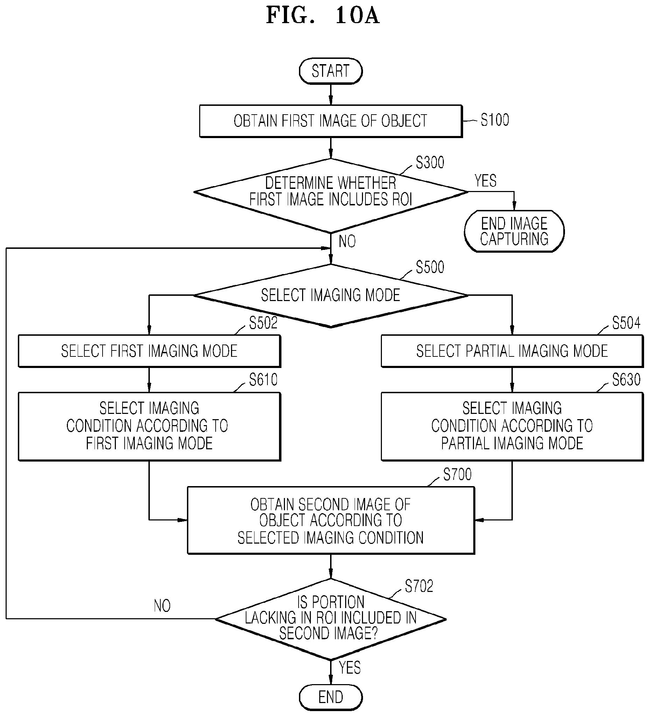

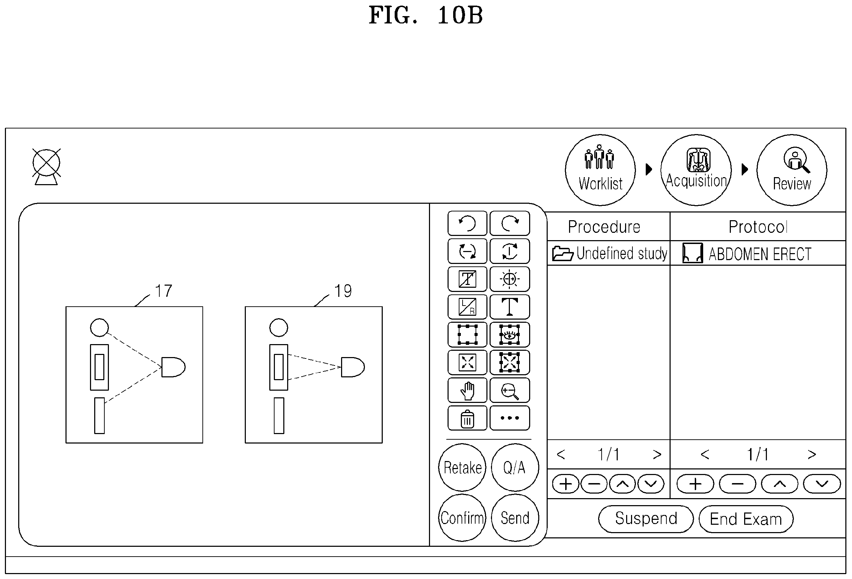

FIGS. 10A and 10B illustrate a method of selecting a imaging mode according to an embodiment of the present invention.

An imaging mode according to the current embodiment of the present invention may include a whole imaging mode A in which the whole ROI of a target object is imaged again and a partial imaging mode B in which a portion of a ROI of a target object is imaged again.

According to the current embodiment of the present invention, as illustrated in FIG. 10A, a first image of a target object may be obtained (S100), and whether the first image includes a ROI or not may be determined (S300), and when the first image includes a ROI, imaging may be ended.

When it is determined that the first image does not include a ROI in operation S300, a imaging mode to reimage the target object may be selected (S500). As described above, the imaging mode according to the current embodiment of the present invention may include the whole imaging mode A and the partial imaging mode B. A imaging mode may be automatically selected by using an X-ray imaging apparatus or may be manually selected via an external input signal input by a user.

For example, when the whole imaging mode A is selected, a imaging condition according to the whole imaging mode may be selected (S610). For example, the imaging condition may include at least one selected from an X-ray radiation intensity, a position of an X-ray source, an amount of collimation (e.g., a range of radiation determined by at least one selected from a position and a size of a collimator), a position of an X-ray detector, and an image resolution.

In operation S700, a second image of the target object may be obtained according to the selected imaging condition in operation S610 described above.

In operation S702, whether there is a portion lacking in the ROI is included in the second image may be determined. If the portion lacking in the ROI is included in the second image, the method may be ended. However, if the portion lacking in the ROI is not included in the second image, the method may return to operation S500 to reselect a imaging mode. Meanwhile, operation S702 may be omitted.

According to an embodiment of the present invention, the partial imaging mode B may be selected.

For example, when the partial imaging mode B is selected, a imaging mode according to the partial imaging mode B may be selected (S630). As described above, the imaging condition may include at least one selected from an X-ray radiation intensity, a position of an X-ray source, an amount of collimation (e.g., a range of radiation determined by at least one selected from a position and a size of a collimator), a position of an X-ray detector, and an image resolution.

In operation S700, a second image of the target object may be obtained according to the imaging condition selected in operation S630.

FIG. 10B illustrates a imaging mode that is set based on an external input signal or the like by a user, according to an embodiment of the present invention.

A user interface may be provided, via which a imaging mode is selected according to an embodiment of the present invention.

A whole imaging mode A and a partial imaging mode B according to an embodiment of the present invention may be provided in the form of a graphic user interface including, for example, an image as illustrated in FIG. 10B. For example, the whole imaging mode A may be displayed as a first image 17 of FIG. 10B. Also, the partial imaging mode B may be displayed as a second image 19 of FIG. 10B. In operation S500, a imaging mode corresponding to an image selected by movement of a cursor or a user's touch (for example, the first image 17 or the second image 19) may be selected. Also, the whole imaging mode A may be provided as a text such as "WHOLE IMAGING" instead of the first image 17 of FIG. 10B, and the partial imaging mode B may also be provided as a text such as "PARTIAL IMAGING" instead of the second image 19 of FIG. 10B.

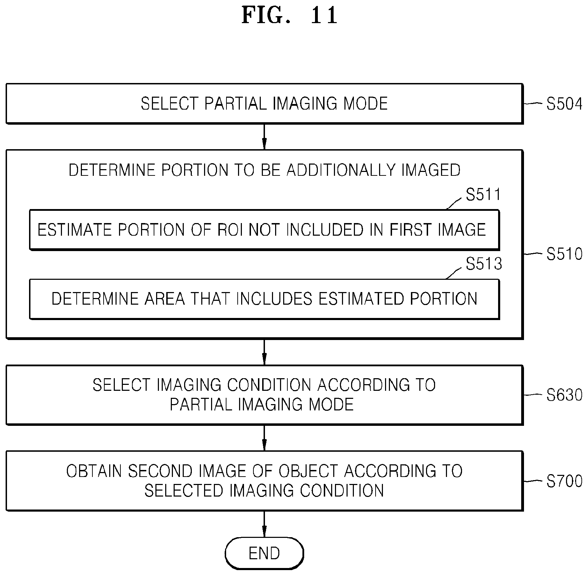

FIG. 11 is a flowchart illustrating a method of determining an additional imaging area when a partial imaging mode is selected, according to an embodiment of the present invention.

The method according to the current embodiment of the present invention may further include determining a portion to be additionally imaged in connection with a ROI (S510), as the partial imaging mode is selected.

A second image according to an embodiment of the present invention may include the portion determined in operation S510.

The determining of a portion to be additionally imaged in connection with a ROI (S510) may include estimating a portion of a ROI that is not included in the first image (S511) and determining an additional imaging area that includes the estimated portion, by using size information of the first image (S513). In this embodiment it is assumed that there are provided two imaging modes: a "whole imaging mode" and a "partial imaging mode".

A imaging condition according to the partial imaging mode may be selected based on the portion that is estimated in operation S510 (S630), and a second image of the target object may be obtained according to the selected imaging condition (S700).

The estimating of a portion of a ROI that is not included in the first image (S511) may include determining a size and a position of the portion not included in the first image based on a size and a position of a ROI included in a standard image (S512).

The size information of the first image according to the current embodiment of the present invention may include at least one of height information and width information of the first image.

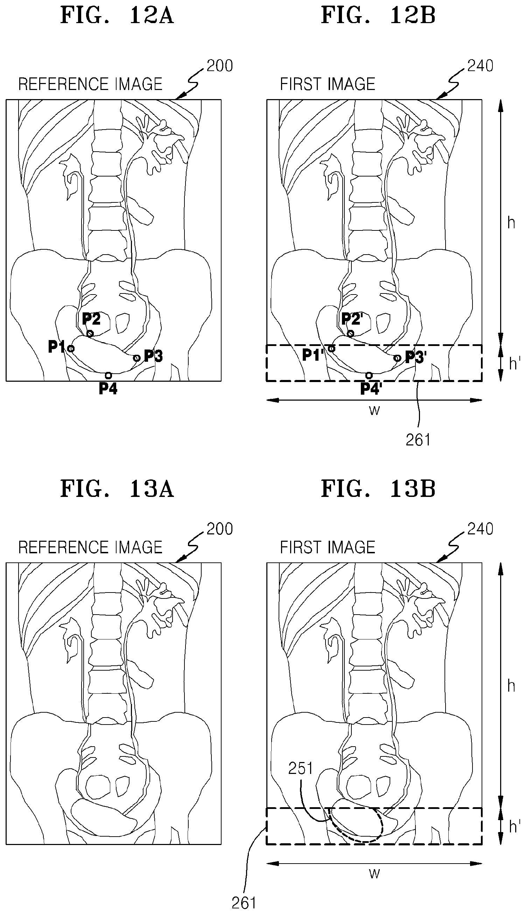

The determining of an additional imaging area (S513) may include determining a imaging area such that all of the ROIs corresponding to the size and the position determined in operation S512 are included in an X-ray image, based on at least one of the height information and the width information of the first image. This will be described later with reference to FIGS. 12 and 13.

FIGS. 12A and 12B illustrate an estimated example of a portion to be additionally imaged, according to an embodiment of the present invention.

FIGS. 13A and 13B illustrate an estimated example of a portion to be additionally imaged, according to another embodiment of the present invention.

FIG. 12A illustrates a standard image 200 of a lower abdominal portion of a target object, and FIG. 12B illustrates a first image 240 of the lower abdominal portion of the target object.

Whether a ROI of the target object is included in the first image 240 may be determined by comparing a plurality of feature points of the standard image 200 (e.g., P1 through P4) and a feature point P1' included in the first image 240.

As illustrated in FIG. 12B, for example, some of a plurality of feature points P1' through P4' corresponding to a plurality of feature points P1 through P4 of a bladder of the standard image are not included in the second image 240, and accordingly, it may be determined that the whole or a part of a bladder is not included in the first image 240.

According to an embodiment of the present invention, a position and a size of a portion not included in the first image 240 (e.g., feature points P1', P3', and P4') of a ROI of the standard image defined by the plurality of feature points P1 through P4 may be estimated.

Also, according to the current embodiment of the present invention, an additional imaging area 261 including the portion estimated in operation S512 may be determined by using size information of the first image 240. For example, the additional imaging area 261 may be determined such that it includes all portions that are estimated as not being included in the first image 240 (e.g., portions defined by the feature points P1', P3', and P4').

In addition, a height and a width of the additional imaging area 261 may be determined based on the size information of the first image 240. In other words, the additional imaging area 261 may be determined such that it includes all portions of a ROI corresponding to the size and the position determined in operation S512 based on at least one of the height information and the width information of the first image 240.

For example, when a height of the first image 240 from an upper end to a lower end thereof in a vertical direction is referred to as a height h, a height h' of the additional imaging area 261 may be determined from a lower limit of the height h of the first image 240 such that the feature point P4 is included in the additional imaging area 261.

Also, when a horizontal size of the first image 240 from the left to the right is referred to as a width w, a width of the additional imaging area 261 may be determined such that the width of the additional imaging area 261 is included in the width w of the first image 240. Alternatively, the width of the additional imaging area 261 may be determined such that it is greater than the width w of the first image 240.

According to another embodiment of the present invention, a portion not included in the first image 240 (e.g., a portion 251 in FIG. 13B) may be estimated by using a boundary line of a ROI.

For example, the portion 251 not included in the first image 240 may be estimated by comparing a boundary line of a ROI included in the standard image 200 and a boundary line obtained from the first image 240.

Also, the additional imaging area 261 including the portion 251 that is lacking in the first image 240 may be determined based on the size information of the first image 240 and the boundary line of a ROI included in the standard image 200. As described above, the additional imaging area 261 including the portion 251 lacking in the first image 240 may be determined by using at least one of the height h and the width w of the first image 240.

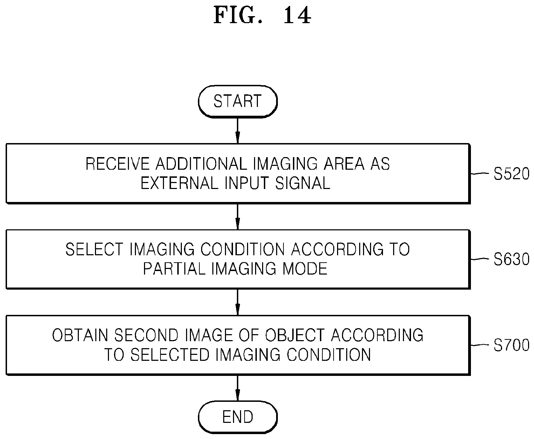

FIG. 14 is a flowchart illustrating a method of determining a portion to be additionally imaged, when a partial imaging mode is selected, according to another embodiment of the present invention.

The method according to the current embodiment of the present invention may further include receiving an additional imaging area of a target object, as an external input signal (S520), when a partial imaging mode B is selected.

In addition, a second image corresponding to the additional imaging area received as an external input signal may be obtained.

The method according to the current embodiment of the present invention may further include setting a imaging condition based on the selected imaging mode (S630). That is, in operation S630, a imaging condition according to the partial imaging mode may be selected based on the additional imaging area received from the outside in operation S520.

The imaging condition may include at least one of, for example, an X-ray radiation intensity, a position of an X-ray source, an amount of collimation (e.g., a range of radiation determined by at least one of a position and a size of a collimator), a position of an X-ray detector, and an image resolution.

Also, obtaining a second image (S700) according to the current embodiment of the present invention may include obtaining an X-ray image of a target object according to the imaging condition set in operation S630. That is, the second image may be obtained according to the imaging condition selected in operation S630.

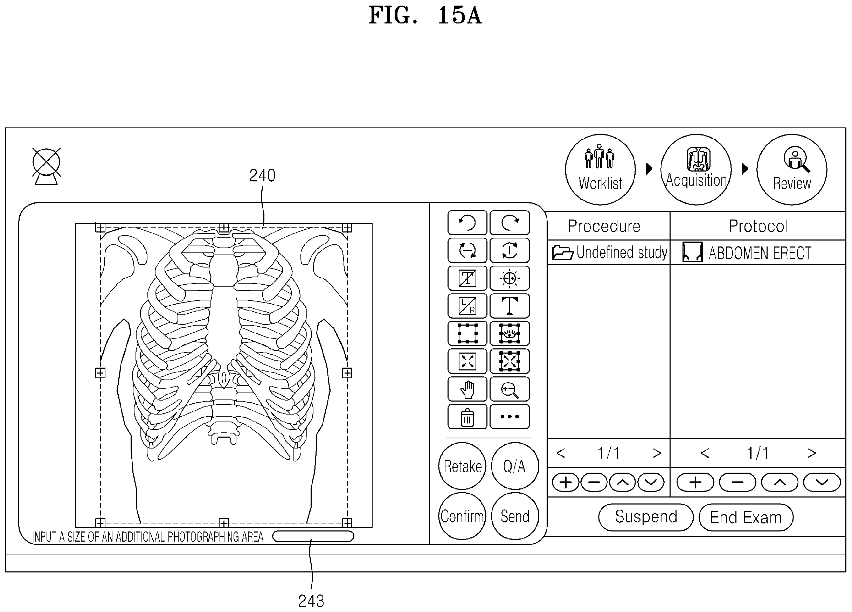

FIGS. 15A through 15C illustrate an example, in which a portion to be additionally imaged is received as an external input signal, according to an embodiment of the present invention.

An interface 243 through which an additional imaging area is to be received may be provided via a screen on which a first image 240 of a target object is displayed. As illustrated in FIG. 15A, the interface 243 may be provided as a form through which a value of an additional imaging area is to be directly input by a user (e.g., a figure input window) or as an image form on which previously set exemplary values of an additional imaging area (e.g., width (w) x height (h), i.e., 14(inch)x1(inch), 14(inch)x2(inch), 12(inch)x1(inch), etc.) including at least one selected from a character, a number, and an icon are included. The previously set exemplary values may be provided to the user in the form of a pull-down menu. A user may select one of the provided values of the pull down menu. For example, a portion to be additionally imaged may be estimated based on a size of a standard image. Also, a portion to be additionally imaged may be determined based on a size of an image that is estimated so that image includes the entire ROI.

In addition, a signal regarding an additional imaging area may be received as sound such as a user's voice, via a microphone or the like.

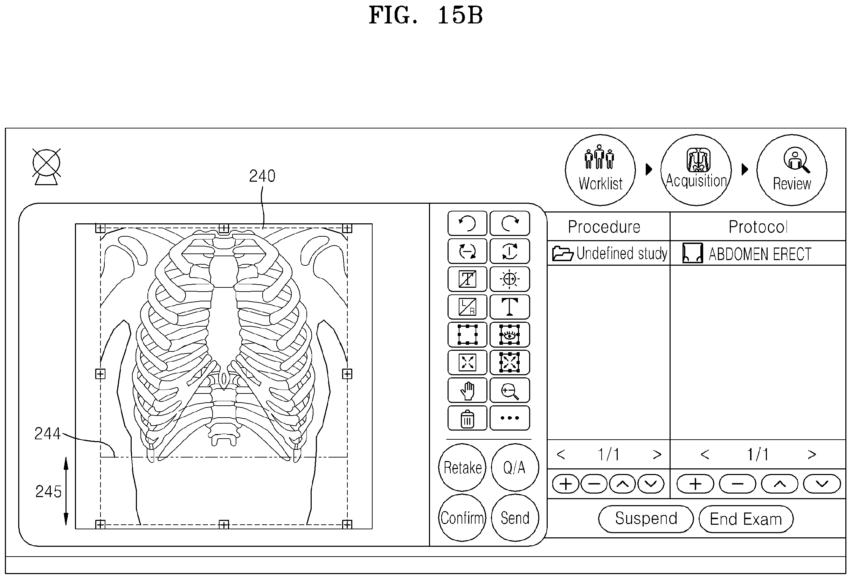

Also, as illustrated in FIG. 15B, the user may set an additional imaging area via an input for manipulating the sliding bar 244 in an upward or downward direction indicated by an arrow 245.

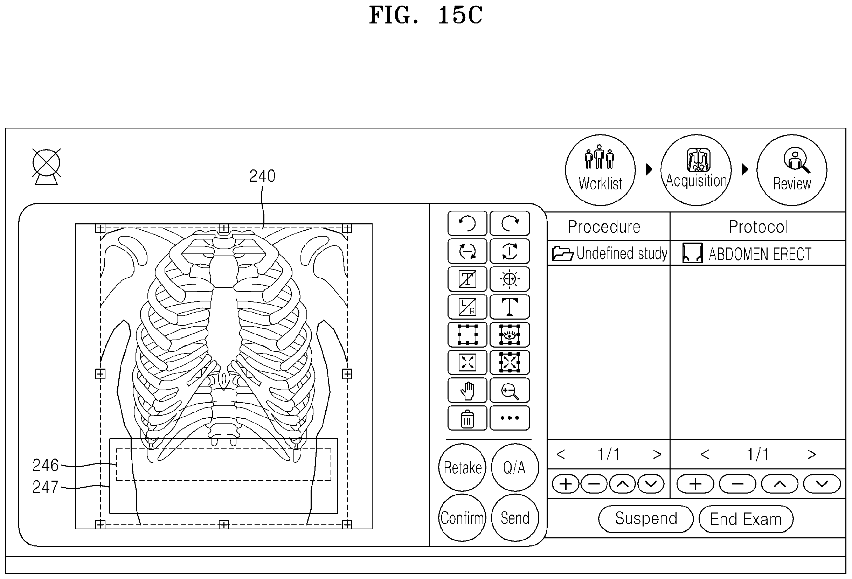

Also, as illustrated in FIG. 15C, the user may set an additional imaging area via an input for reducing or extending a size of an adjustable window 246 which may be larger or smaller than the additional imaging area. Also, in order to obtain a precise second image including a portion that is lacking in the first image 240, an area that is to be potentially overlapped, for example, an area 247, may be extended. For example, the area 247 may be extended on a screen that displays the first image 240 illustrated in FIGS. 15A through 15C. Also, the area 247 may be extended by being popped up on the screen that displays the first image 240 or by being displayed on another screen as a different layer so that the user may easily observe the area 247.

Although an additional imaging area is illustrated in a lower portion of the first image 240 in FIGS. 15A through 15C, the embodiments of the present invention are not limited thereto. For example, the additional imaging area may be near an upper portion of the first image 240. In other words, as illustrated in FIGS. 15A through 15C, the portion of the ROI lacking in the first image 240 may be an upper portion or a lower portion of the first image 240.

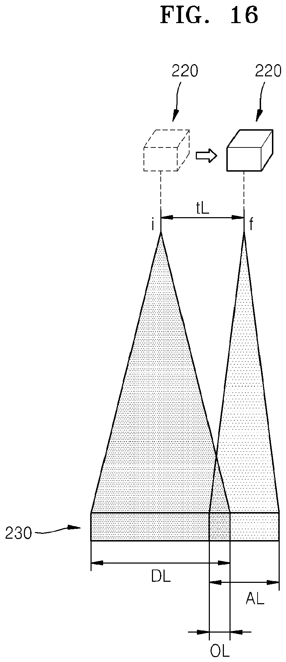

FIG. 16 illustrates an example of selecting a imaging condition, according to an embodiment of the present invention.

When an X-ray source 220 according to an embodiment of the present invention is a stepping type, a imaging condition regarding a position of the X-ray source 220 may be selected as follows. The X-ray source of a stepping type refers to the X-ray source moving along the predetermined virtual moving line to image a target object.

An X-ray irradiated at a first position i of the X-ray source 220 may be detected by using an X-ray detector 230 to obtain a first image. As described above, the X-ray source 220 may move to a second position f to correspond to an additional imaging area that is estimated according to an embodiment of the present invention or that is determined based on a value received from the outside.

A distance tl that the X-ray source 220 moves may be obtained based on Equation 1 below.

$$tl = (dl + AL)/2 - ol \hspace{3cm} [\text{Equation 1}]$$

The distance tl that the X-ray source 220 has moved corresponds to a difference between the second position f and the first position i, and a length dl of the X-ray detector 230 refers to a length of the X-ray detector 230 that is used in obtaining a first image, and an additional imaging area AL refers to a length corresponding to a imaging area to obtain a second image, and an overlapped length ol may refer to a length by which the first and second images are overlapped.

The overlapped length ol according to the current embodiment of the present invention may have various values according to ROIs. For example, an amount of exposure to radiation with respect to the target object may be minimized by optimizing the overlapped length according to relative template sizes of respective imaged areas. The overlapped length ol according to the current embodiment of the present invention may be included in a range from, for example, about 35 mm to about 90 mm in consideration of a tube anode heel effect of the X-ray source 220. The overlapped length ol may be 50 mm.

For example, according to the current embodiment of the present invention, the overlapped length ol may be set to be about 4% of a height value of the second image. For example, when the height value of the second image is 1125 mm, the overlapped length ol may be 45 mm. The overlapped length ol may be set such that a cumulative dose of radiation with respect to the target object is minimized.

Also, according to an embodiment of the present invention, to obtain a second image, the X-ray detector 230 may move according to the additional imaging area AL in one of an upward direction, a downward direction, a direction to the left, a direction to the right, and a diagonal direction. In addition, at least one selected from the X-ray source 220 and the X-ray detector 230 may be moved automatically or manually according to a selected imaging mode.

Also, the X-ray source 220 may be a stationary X-ray source, and the target object may be located on a table (not shown). The table may be moved by using a driving mechanism (not shown) so as to obtain a predetermined portion of the target object (for example, a lacking portion of a ROI). An X-ray detector may be embedded in the table or disposed under the table. Also, the X-ray detector may be disposed separately from the table or may be moved automatically or manually according to a selected imaging mode.

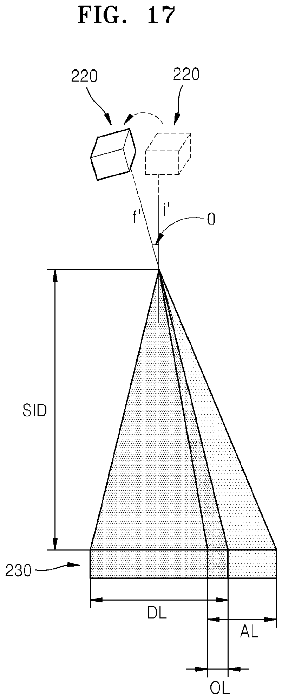

FIG. 17 illustrates an example of selecting a imaging condition, according to another embodiment of the present invention.

When an X-ray source 220 according to an embodiment of the present invention is a rotation type, a imaging condition regarding a position of the X-ray source 220 may be selected as follows. The X-ray source of a rotation type refers to the X-ray source rotating on the basis of the predetermined virtual point to image a target object.

An X-ray irradiated at a first position i' of the X-ray source 220 may be shown as a first image through the X-ray detector 230. The X-ray source 220 may be rotated by a predetermined angle ($\theta$) to be moved to a second position f' so as to correspond to an additional imaging area that is estimated according to an embodiment of the present invention or that is determined based on a value received from the outside, as described above. In this case, the rotational angle ($\theta$) of the X-ray source 220 may refer to an angle between the second position f' and the first position i'.

The rotational angle (θ) of the X-ray source 220 may be obtained based on Equations 2 and 3 below.

$$\theta = \tan^{-1}\left(\frac{IL}{SID}\right)$$

[Equation 2]

$$IL = (dl + AL)/2 - ol \qquad \text{[Equation 3]}$$

IL may refer to a rotational movement distance of the X-ray source 220 formed by rotating the X-ray source 220, and SID may refer to a distance between the X-ray source 220 and an X-ray image. An X-ray image is obtained from data detected by using the X-ray detector 230, and thus, SID may include, for example, a distance between the X-ray source 220 and the X-ray detector 230.

Also, an X-ray irradiation angle according to rotation of the X-ray source 220 may be included in a range from about 12% to about 15% with respect to the whole allowable range of the X-ray irradiation angle. The X-ray irradiation angle may be adjusted to be about 12% with respect to the whole allowable range of the X-ray irradiation angle.

Also, a length dl of the X-ray detector 230 may refer to a length of the X-ray detector 230 used in obtaining the first image, and an additional imaging area AL may refer to a length corresponding to a imaging area to obtain the second image, and an overlapped length ol may refer to a length by which the first and second images are overlapped.

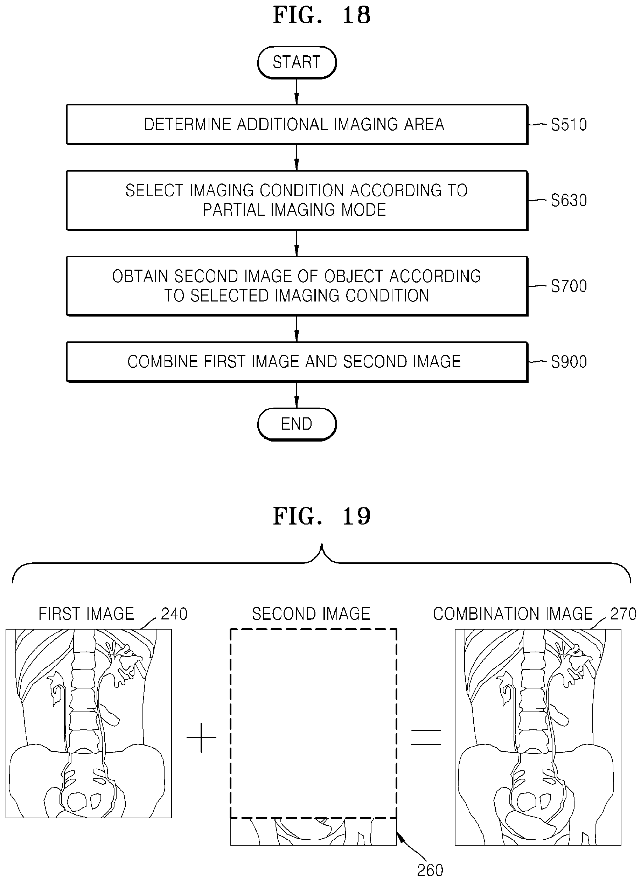

The overlapped length ol according to the current embodiment of the present invention may have various values according to ROIs. For example, an amount of exposure to radiation with respect to the target object may be minimized by optimizing the overlapped length according to relative template sizes of respective imaged areas. The overlapped length ol according to the current embodiment of the present invention may be included in a range from, for example, about 35 mm to about 90 mm in consideration of a tube anode heel effect of the X-ray source 220. The overlapped length ol may be 50 mm. For example, according to the current embodiment of the present invention, the overlapped length ol may be set to be about 4% of a height value of the second image. For example, when the height value of the second image is 1125 mm, the overlapped length ol may be 45 mm. The overlapped length ol may be set such that a cumulative dose of radiation with respect to the target object is minimized. FIG. 18 is a flowchart illustrating a method of combining a first image and a second image, according to an embodiment of the present invention.

The method according to the current embodiment of the present invention may further include combining the first image and the second image.

For example, when a partial imaging mode B is selected, an additional imaging area may be determined (S510), and a imaging condition according to the partial imaging mode may be selected based on the determined additional imaging area (S630), and a second image of a target object may be obtained according to the selected imaging condition (S700). Also, the first image and the second image may be combined by using a predetermined image combining method (S900). This will be described later with reference to FIGS. 20A and 20B.

FIG. 19 illustrates an example of combining a first image 240 and a second image 260, according to an embodiment of the present invention.

A combined image 270 may be obtained by applying a predetermined image combining technique to the first image 240 and the second image 260. As described above, the combined image 270 may be an image including an ROI of a target object.

FIGS. 20A and 20B illustrate an example of combining a first image 240 and a second image 260, according to another embodiment of the present invention.

According to the current embodiment of the present invention, images may be combined by using at least one template. Matching of a plurality of images refers to stitching an image (for example, a reference image) and another image (for example, a stitching image) together. A search area may be provided in the reference image, and a template may be provided in the stitching image. In other words, matching of a plurality of images may refer to a process of determining a matching condition of a stitching image with respect to a reference image (for example, determining a movement distance of X coordinates or Y coordinates of an image or a rotational angle of an image). For example, a temperate such as a template T100 refers to a predetermined area in a stitching image, for which a condition for matching with respect to a plurality of images is searched for, and may have various shapes such as a template to which weights are applied to respective major ROIs or a single template. A matching condition such as a movement distance or an angle of a stitching image with respect to a reference image may be searched for by using the template to match the plurality of images.

According to the current embodiment of the present invention, images may be combined by using at least one template.

A template (e.g., a template T100) may refer to an area defined by a predetermined position or a predetermined size in an image of a target object. Also, the template (e.g., a template T100) may refer to the whole or a portion an image of a target object. The template (e.g., a template T100) may be used as a reference area for stitching a plurality of images.

The template T100 having a predetermined size at a predetermined position of the first image 240 may be set. A combined image 270 may be obtained by performing template matching with respect to the first image 240 and the second image 260 based on the template T100.

For example, when the template T100 having a predetermined size at a predetermined position of the first image 240 is set, a substantially identical portion corresponding to the template T100 may be detected from the second image 260. For example, a substantially identical portion included in the second image 260 that has a large similarity to the template T100 may be detected from the second image 260.

Also, a second image according to an embodiment of the present invention may include at least one template to be used in combining the second image with a first image. The at least one template included in the second image may correspond to at least one template with respect to the first image. For example, at least one template set with respect to the second image may be matched to the at least one template set with respect to the first image.

The combining of the first and second images according to the current embodiment of the present invention (S900) may include stitching (or matching) the second image to the first image by using at least one template.

For example, the first and second images may be stitched based on at least one template with respect to the first image or based on at least one template with respect to the second image. Alternatively, the first and second images may be stitched by using respective templates of the first and second images.

Also, a position and a size of at least one template according to the current embodiment of the present invention may be set in various ways. That is, at least one template according to an embodiment of the present invention may be set differently based on at least one of properties of a target object, a size of a ROI, and a position of a ROI.

The properties of the target object according to an embodiment of the present invention may include the age, gender, height, weight, body type, a part to be imaged, and medical history of the target object.

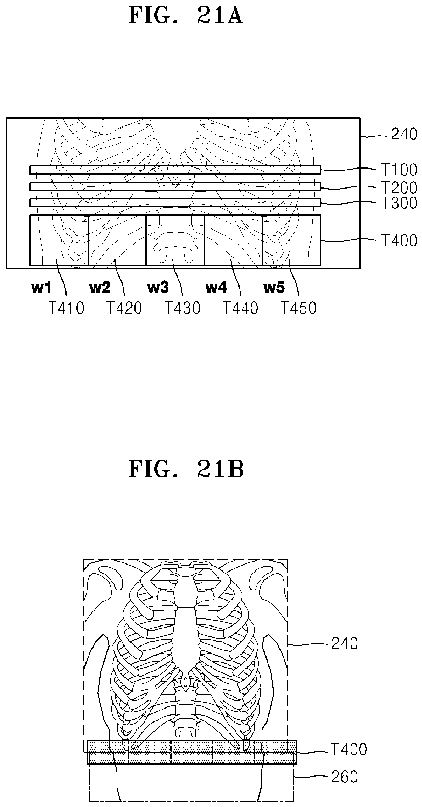

Also, the second image according to the current embodiment of the present invention may include a plurality of templates to be used in combining the second image with the first image. The combining of the first and second images (S900) may include applying different weights to a plurality of templates and stitching the first and second images based on the applied weights. This will be described below with reference to FIGS. 21A and 21B.

FIGS. 21A and 21B illustrate an example of combining a first image and a second image, according to another embodiment of the present invention.

According to an embodiment of the present invention, a plurality of different templates T100 through T400 may be set with respect to a first image 240. Also, similarly, a plurality of different templates may be set with respect to a second image 260.

A template in the form of a block that has a fixed size and is set at a fixed position may be vulnerable to a reversion phenomenon between imaged images, particularly, in imaging a target object using an X-ray source of a stepping type, and thus, it may be difficult to obtain an exact X-ray image of a target object. Also, when a template in the form of a block that has a fixed size and is set at a fixed position is used, if the target object moves or image quality of an obtained image of the target object is not uniform, efficiency of image stitching may decrease.

According to the current embodiment of the present invention, at least one of a position and a size of a template may be variably set based on a feature point or a boundary line of a ROI of a target object. For example, at least one of a position and a size of a template may be set such that the template includes a feature point of a ROI of a target object included in the first image 240 (e.g., a feature point P2' of FIG. 12B). That is, at least one of a position and a size of a template may be set such that the template necessarily includes a feature point included in the first image 240 (e.g., a feature point P2' of FIG. 12B).

Also, the template according to the current embodiment of the present invention may be set to have a predetermined size at a position that is nearest to the feature point included in the first image 240 (e.g., a feature point P2' of FIG. 12B), but is not limited thereto.

Also, as illustrated in FIG. 21A, the template T400 according to the current embodiment of the present invention may include at least one sub-template (e.g., T410 through T450).

According to an embodiment of the present invention, different weights may be applied to a plurality of sub-templates (e.g., T410 through T450) during operation S900 of FIG. 18.

For example, different weights may be respectively applied to sub-templates, such that a weight W1 is applied to a template T410, and a weight W2 is applied to a template T420. For example, weights W1 through W5 applied to a plurality of sub-templates may be different from one another. Also, some of sub-templates may have the same weight, which is different from those of other sub-templates (for example, W1=W4=W5 and W1<W2<W3), but embodiments of the present invention are not limited thereto.

According to an embodiment of the present invention, by using the plurality of templates T410 through T450 to which weights are applied, the first image 240 and the second image 260 may be stitched. Image stitching is performed based on the above-described applied weights so that a data amount to be processed during image stitching may be reduced, and thus, a combination image may be quickly obtained, and a imaging time may be reduced.

For example, elements of an image area (e.g., a pixel value) included in a template to which a relatively high weight (e.g., W3 in the above-described example) is applied may be used frequently in image stitching. On the other hand, elements of an image area included in a template to which a relatively low weight is applied may be considered relatively little in image stitching.

The first image 240 and the second image 260 may be stitched smoothly and naturally through a blending operation. Blending between images may include an operation of adjusting a mixing ratio between a first image and a second image near an image stitching portion to express the image stitching portion smoothly and naturally.

Also, a series of processes to appropriately match the first image 240 and the second image 260 may be additionally performed by cropping a portion or the whole of each of the first and second images 240 and 260 or rotating the first and second images 240 and 260 or adjusting phases of the first and second images 240 and 260.

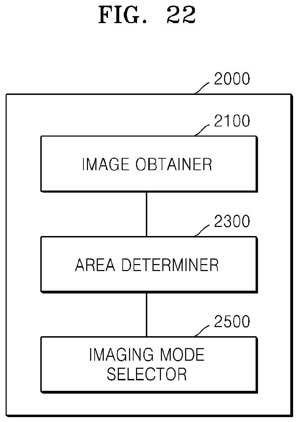

FIG. 22 is a block diagram illustrating an apparatus 2000 for obtaining an X-ray image including a ROI of a target object according to an embodiment of the present invention.

The apparatus 2000 for obtaining an X-ray image of a ROI of a target object may include an image obtaining unit 2100 for obtaining a first image of a target object, and a imaging mode providing unit 2400 configured for providing a plurality of imaging modes, preferably corresponding with imaging areas having different sizes. Further there may be provided an area determining unit 2300 for determining whether the first image includes a ROI, and a imaging mode selecting unit 2500 for selecting a imaging mode of said plurality of imaging modes to reimage a target object based on a result of determination by the area determining unit 2300. Note that the area determining unit 2300 may be omitted in an embodiment where the user determines himself which portion of the ROI is missing, and that the imaging mode selecting unit 2500 may be an interface allowing the user to select a imaging mode of the plurality of imaging modes. In a preferred automated embodiment the area determining unit may be configured for determining a portion of the ROI which is missing in the first image, and the imaging mode selecting unit may be configured for selecting a imaging mode of the plurality of imaging modes corresponding with a imaging area having the smallest size capable of containing the portion which is missing in the first image.

The image obtaining unit 2100 may obtain a first X-ray image 240 of a target object 210 located between an X-ray source 220 and an X-ray detector 230. For example, while a user intends to obtain a first X-ray image 240 including both a lung and a bladder of a target object, all or a portion of a bladder 250 may not be included in the first X-ray image 240. For example, the first image may be analyzed in order to reduce a degree of exposure of the target object to radiation, and a imaging mode may be selected, in which a second image including a portion lacking in the first image is imaged. The above operations may be performed on a target object existing at a predetermined position, and the first image of the target object existing at the predetermined position may be a standard for selecting a imaging mode.

Also, the imaging mode may be selected from among a plurality of imaging modes corresponding to imaging areas having different sizes. The plurality of imaging modes may include a whole imaging mode and a partial imaging mode.

The area determining unit 2300 may determine whether a ROI is included in a first image by comparing a standard image of a target object and the first image. An operation of comparing images according to the current embodiment of the present invention may include a series of operations to determine similarity between a plurality of images.

The imaging mode providing unit 2400 is configured for providing a plurality of imaging modes, and the imaging mode selecting unit 2500 may select a imaging mode of this plurality of imaging modes to reimage a target object based on a result of the determination by the area determining unit 2300.

Also, according to the current embodiment of the present invention, a second image of the target object may be obtained by using the image obtaining unit 2100 according to the imaging mode selected by using the imaging mode selecting unit 2500, and the second image may include a portion or the whole of a ROI.

The area determining unit 2300 according to the current embodiment of the present invention may compare the standard image of the target object and the first image.

The standard image may be selected from among previously stored images based on properties of the target object. The standard image of the target object may be statistically determined according to a portion of the target object and stored in a database in advance.

For example, predetermined elements found in X-ray images of a chest of a plurality of target objects (e.g., the number of ribs or positions of diaphragms included in the images) are extracted, and the plurality of apparent elements may be defined as common features based on a frequency with which predetermined elements extracted from the X-ray images of the chest of the plurality of target objects appear, and the X-ray images of the chest of the target objects including the common features may be defined as standard images and stored.

In addition, the standard images according to the current embodiment of the present invention may be selected from among images of the plurality of target objects stored in advance, based on properties of a target object that is to be imaged. The properties of the target object according to an embodiment of the present invention may include the age, gender, height, weight, body type, part to be imaged, and medical history of the target object that is to be imaged.

In other words, a standard image may be selected from among images of a plurality of target objects that are stored in advance, based on at least one of the age, gender, height, weight, body type, a part to be imaged, and medical history of the target object that is to be imaged. For example, from among the images of a plurality of target objects that are stored in advance, a standard image of a chest of a child and that of a chest of an adult may be selected based on different elements. Also, as illustrated in FIGS. 5A through 5E, standard images may be differently selected according to a part to be imaged of a target object.

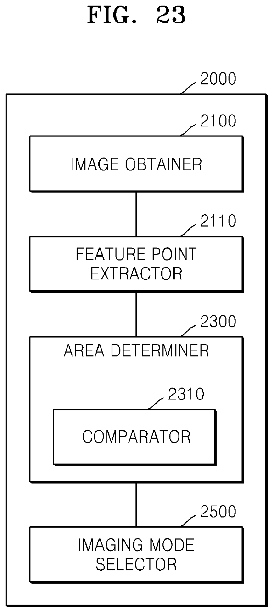

FIG. 23 is a block diagram illustrating an apparatus 2000 for obtaining an X-ray image including a ROI of a target object, according to another embodiment of the present invention.

The apparatus 2000 according to the current embodiment of the present invention may further include a feature point extracting unit 2110 for extracting at least one feature point related to a ROI, from an obtained first image, and an area determining unit 2300 may further include a comparing unit 2310 comparing a feature point extracted from the first image by using the feature point extracting unit 2110 with a feature point of a ROI included in a standard image.

Whether the first image includes a ROI or not may be determined by using a SIFT method in which a feature point of a target object is used or by using a model-based matching method.

The number and a position of at least one feature point may be defined in advance according to a size and a position of an ROI according to an embodiment of the present invention.

The feature point according to an embodiment of the present invention may include a predetermined point to distinguish, for example, tissues included in an X-ray image of a target object. For example, the feature point may be used as an identifier to identify at least one of a shape, size, and position of a tissue or the like included in an X-ray image of a target object.

As illustrated in FIG. 7A again, for example, a plurality of feature points (e.g., feature points p1 through p5) may indicate that a imaged tissue is a skull of a target object. That is, the skull of the target object may be identified in an X-ray image based on a plurality of feature points (e.g., feature points P1 through P5).

Also, referring to FIGS. 7B through 7E again, at least one feature point of each portion of a target object may be determined in advance.

The comparing unit 2310 may compare the number and a position of a feature point of the ROI included in the standard and the number and a position of a feature point extracted from the first image. The area determining unit 2300 may determine whether the first image includes a ROI of a target object based on a result of determination by the comparing unit 2310.

For example, when a skull of a target object is an ROI, and only some feature points (e.g., P1', P2', and P5') are found in the first image that is obtained in order to capture an image of the skull of the target object, feature points (e.g., P3' and P4') of the first image corresponding to the feature points P3 and P4 from among a plurality of feature points (e.g., P1 through P5) included in the standard image do not exist. Thus, it may be determined that the first image does not include a ROI. That is, a lower jaw corresponding to the feature points P3 and P4 of the standard image may be determined as being not included in the first image.

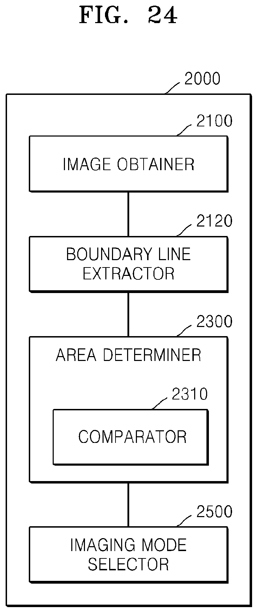

FIG. 24 is a block diagram illustrating an apparatus for obtaining an X-ray image including a ROI of a target object, according to another embodiment of the present invention.

The apparatus 2000 may further include a boundary line extracting unit 2120 extracting a boundary line of a ROI from an obtained first image and a comparing unit 2310 comparing the boundary line extracted from the first image with a boundary line of a ROI included in a standard image.

Similarly to the feature points, the boundary line according to the current embodiment of the present invention may include a predetermined line used to distinguish, for example, a tissue included in an X-ray image of a target object. The line may be a solid line, a dotted line, or an alternating long and short dashed line, but is not limited thereto. The boundary line may be used as an identifier to identify at least one of a shape, size, and position of the tissue included in the X-ray image of the target object.

Referring to FIG. 9A again, for example, a skull of a target object may be identified by a boundary line l1. Also, as illustrated in FIGS. 9B through 9E, a boundary line of each portion of the target object may be edited and may be determined in advance.

The comparing unit 2310 may determine a similarity between the boundary line extracted from the first image and the boundary line of the ROI included in the standard image. The area determining unit 2300 may determine whether the fist image includes a ROI of the target object based on a result of the determining of the similarity by the comparing unit 2310.

For example, the similarity between two boundary lines l1 and l1' may be determined based on whether a boundary line (e.g., l1') extracted from the first image is cut or deformed compared to a boundary line (11) of the ROI included in the standard image, and accordingly, whether the first image includes the ROI or not may be determined.

For example, when a similarity between the boundary line (e.g., I1) of the ROI included in the standard image and the boundary line (e.g., I1') extracted from the first image is about 95% or greater, it may be determined that the ROI is included in the first image, and when the similarity is less than about 75%, it may be determined that the ROI is not included in the first image. However, a value of the similarity used as a reference for the above determination is not limited thereto.

The imaging mode providing unit 2400 may be configured for providing a whole imaging mode in which the entire ROI of a target object is reimaged and a partial imaging mode in which a portion of a ROI of a target object is reimaged. The imaging mode selecting unit 2500 may select one of the whole imaging mode in which the entire ROI of a target object is reimaged and the partial imaging mode in which a portion of a ROI of a target object is reimaged.

Referring to FIG. 10 again, a first image of a target object may be obtained by using the image obtaining unit 2100, and whether the first image includes a ROI may be determined by using the area determining unit 2300, and when the first image includes a ROI, imaging may be ended.

Also, when the area determining unit 2300 determines that the first image does not include a ROI, a imaging mode for reimaging a target object may be selected by using the imaging mode selecting unit 2500. The imaging mode may include the whole imaging mode A and the partial imaging mode B.

For example, when the whole imaging mode A is selected, a imaging condition according to the whole imaging mode may be selected. The imaging condition may include at least one of, for example, an X-ray radiation intensity, a position of an X-ray source, an amount of collimation (e.g., a range of radiation determined by at least one of a position and a size of a collimator), a position of an X-ray detector, and an image resolution.

Also, a second image of the target image may be obtained by using the image obtaining unit 2100 according to the imaging condition selected by using the imaging condition setting unit 2800.

According to an embodiment of the present invention, the partial imaging mode B may be selected.

For example, when the partial imaging mode B is selected, a imaging condition according to the partial imaging mode may be selected by using the imaging condition setting unit 2800. As described above, the imaging condition may include at least one of, for example, an X-ray radiation intensity, a position of an X-ray source, an amount of collimation (e.g., a range of radiation determined by at least one of a position and a size of a collimator), a position of an X-ray detector, and an image resolution.

Also, a second image of the target object may be obtained by using the image obtaining unit 2100 according to the imaging condition selected by using the imaging condition setting unit 2800.

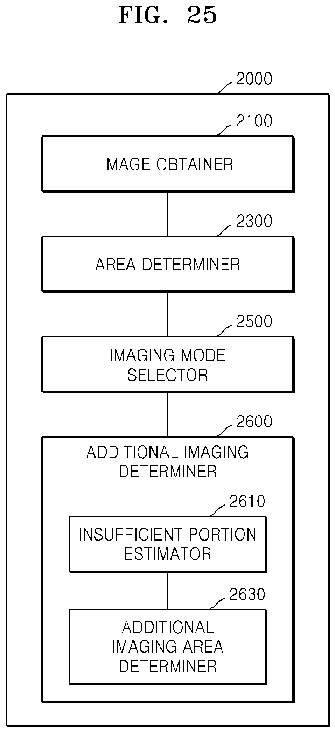

FIG. 25 is a block diagram illustrating an X-ray image obtaining apparatus 2000 that further includes an additional imaging determining unit according to an embodiment of the present invention.

The apparatus 2000 may further include an additional imaging determining unit 2600 that determines a portion of a ROI that is to be additionally imaged, as a partial imaging mode is selected by using the imaging mode selecting unit 2500. A second image including the portion determined by using the additional imaging determining unit 2600 may be obtained.

The additional imaging determining unit 2600 according to the current embodiment of the present invention may include an insufficient portion estimating unit 2610 that estimates a portion of a ROI not included in the first image and an additional imaging area determining unit 2630 that determines an additional imaging area including the portion estimated by using the insufficient portion estimating unit 2610 by using size information of the first image.

The insufficient portion estimating unit 2610 may determine a size and a position of a portion not included in the first image based on a size and a position of a ROI included in a standard image.

The size information of the first image according to the current embodiment of the present invention may include at least one of height information and width information of the first image.

The additional imaging area determining unit 2630 may determine a imaging area such that all of portions of a ROI corresponding to the size and the position of the portion determined by using the insufficient portion estimating unit 2610 are included in the additional imaging area, based on at least one of the height information and the width information of the first image.

Referring to FIGS. 12A and 12B again, FIG. 12A illustrates a standard image 200 of a lower abdominal portion of a target object, and FIG. 12B illustrates a first image 240 of the lower abdominal portion of the target object.

Whether a ROI of the target object is included in the first image 240 may be determined by comparing a plurality of feature points of the standard image 200 (e.g., P1 through P4) and a feature point P1' included in the first image 240. As illustrated in FIG. 12B, for example, when some (P1', P3', and P4') of a plurality of feature points P1' through P4' corresponding to a plurality of feature points P1 through P4 of a bladder of the standard image are not included in the second image 240, it may be determined that the whole or a partial image of the bladder is not included in the first image 240.

According to an embodiment of the present invention, a position and a size of a portion not included in the first image 240 (e.g., a portion defined by feature points P1', P3', and P4') in a ROI defined by the plurality of feature points P1 through P4 may be estimated in the standard image.

Also, according to the current embodiment of the present invention, an additional imaging area 261 (see figure 13B) including the portion estimated by using the insufficient portion estimating unit 2610 may be determined by using the additional imaging area determining unit 2630 by using size information of the first image 240. For example, the additional imaging area 261 may be determined such that all portions that are estimated as not being included in the first image 240 (e.g., a portion defined by feature points P1', P3', and P4') are included in the additional imaging area 261.

In addition, a height and width of the additional imaging area 261 may be determined based on the size information of the first image 240. In other words, the additional imaging area 261 may be determined by using the additional imaging area determining unit 2630 such that all portions of a ROI corresponding to the size and position of the portion determined by using the insufficient portion estimating unit 2610 are included in the additional imaging area 261 based on at least one of the height information and the width information of the first image 240.

For example, when a height of the first image 240 from an upper end to a lower end thereof in a vertical direction is referred to as h, a height h' of the additional imaging area 261 may be determined from a lower limit of the height h of the first image 240 such that the feature point P4' is included.

Also, when a horizontal size of the first image 240 from the left to the right is referred to as a width w, a width of the additional imaging area 261 may be determined such that the width of the additional imaging area 261 is included in the width of the first image 240. Alternatively, the width of the additional imaging area 261 may be determined such that it is greater than the width of the first image 240.

According to another embodiment of the present invention, referring to FIGS. 13A and 13B, a portion not included in the first image 240 (e.g., a portion 251 in FIG. 13B) may be estimated by using a boundary line of a ROI.

For example, the portion 251 not included in the first image 240 may be estimated by comparing a boundary line of a ROI included in the standard image 200 and a boundary line obtained from the first image 240.

Also, the additional imaging area 261 including the portion 251 that is lacking in the first image 240 may be determined based on the size information of the first image 240 and the boundary line of a ROI included in the standard image 200. As described above, the additional imaging area 261 including the portion 251 lacking in the first image 240 may be determined by using at least one of the height h and the width w of the first image 240.

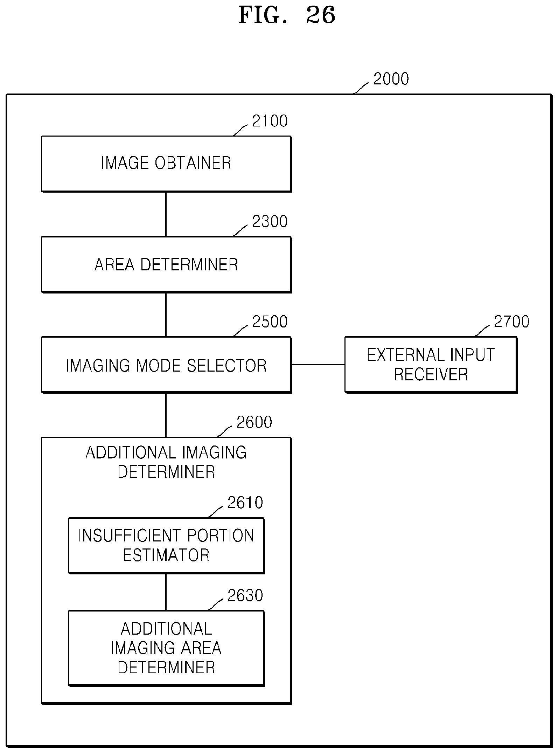

FIG. 26 is a block diagram illustrating an X-ray image obtaining apparatus 2000 that further includes an external input receiving unit and an additional imaging determining unit, according to an embodiment of the present invention.

The apparatus 2000 may further include an external input receiving unit 2700 that receives an additional imaging area of a target object, as an external input signal, as a partial imaging mode is selected by using the imaging mode selecting unit 2500. A second image corresponding to the additional imaging area received as an external input signal may be obtained.

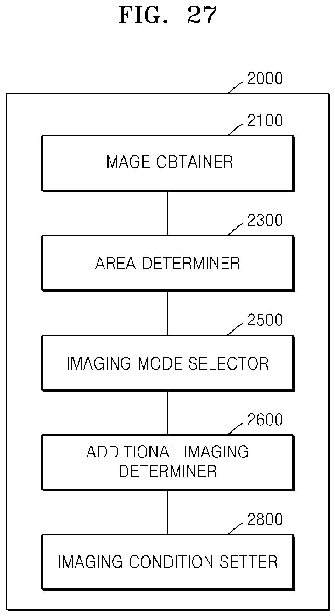

FIG. 27 is a block diagram illustrating an X-ray image obtaining apparatus 2000 that further includes a imaging condition setting unit 2800, according to an embodiment of the present invention.

The apparatus 2000 may further include the imaging condition setting unit 2800 that sets a imaging condition based on a imaging mode selected by using the imaging mode selecting unit 2500. The second image may be obtained as an X-ray image of a target object according to a imaging condition set by using the imaging condition setting unit 2800.

According to an embodiment of the present invention, a imaging condition according to a partial imaging mode may be selected based on an additional imaging area received from the outside by using the external input receiving unit 2700.

The imaging condition according to an embodiment of the present invention may include at least one of a position of an X-ray source, an amount of collimation, a position of an X-ray detector, and an image resolution.

That is, the imaging condition may include at least one of, for example, an X-ray radiation intensity, a position of an X-ray source, an amount of collimation (e.g., a range of radiation determined by at least one of a position and a size of a collimator), a position of an X-ray detector, and an image resolution.

Referring to FIG. 15 again, for example, an interface 243 through which an additional imaging area is to be received may be provided via a screen on which a first image 240 of a target object is displayed. As illustrated in FIG. 15, the interface 243 may be provided in a form through which a value of an additional imaging area may be directly input by a user (e.g., a figure input window) or in an image form on which previously set exemplary values of an additional imaging area (e.g., width x height, i.e., 14(inch)x1(inch), 14(inch)x2(inch), 12(inch)x1(inch), etc.) including at least one of a character, a number, and an icon are included. In other words, the external input receiving unit 2700 may receive an external input that is input via the interface 243 as illustrated in FIG. 15.

In addition, a signal regarding an additional imaging area may be received as sound such as the user's voice, via a microphone or the like.

When an X-ray source 220 according to an embodiment of the present invention is of the stepping type, for example, a imaging condition for a position of the X-ray source 220 may be obtained with reference to FIG. 16 and Equation 1. Also, when the X-ray source 220 is of the rotation type, for example, a imaging condition for a position of the X-ray source 220 may be obtained with reference to FIG. 17 and Equations 2 and 3.

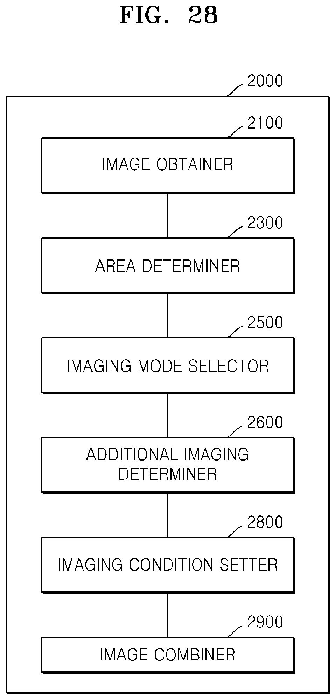

FIG. 28 is a block diagram illustrating the X-ray image obtaining apparatus 2000 that further includes an image combining unit 2900, according to an embodiment of the present invention.

The apparatus 2000 may further include the image combining unit 2900 that combines an obtained first image and an obtained second image.

For example, when the partial imaging mode B is selected, an additional imaging area may be determined by using the additional imaging determining unit 2600, and a imaging condition according to the partial imaging mode is selected by using the imaging condition setting unit 2800 based on the determined additional imaging area. The image combining unit 2900 may combine the second image and the first image obtained by using the image obtaining unit 2100 according to the selected imaging condition by using a predetermined image combining method.

According to an embodiment of the present invention, images may be combined by using at least one template.

A template (e.g., a template T100 of FIGS. 20A and 20B) may refer to an area defined by a predetermined position or a predetermined size in an image of a target object. Also, the template (e.g., a template T100) may refer to a predetermined image of the target object. The template may be used as a reference area used when stitching a plurality of images.

The image combining unit 2900 may set the template T100 having a predetermined size at a predetermined position of a first image 240. A combined image 270 may be obtained by performing template matching with respect to the first image 240 and the second image 260 based on the template T100.

For example, when the template T100 having a predetermined size at a predetermined position of the first image 240 is set, a portion of the first image 240 corresponding to the template T100 may be detected from the second image 260. For example, a portion of the first image 240 that has a large similarity with respect to the template T100 may be detected from the second image 260.

A first image according to an embodiment of the present invention may include at least one template to be used in combining the first image with a second image. Also, the second image may include at least one template to be used in combining the second image with the first image.

As described above, at least one template included in the second image may correspond to at least one template with respect to the first image. For example, at least one template with respect to the second image may be set to match at least one template set with respect to the second image.

The image combining unit 2900 may stitch an obtained second image with an obtained first image by using at least one template.

For example, the first and second images may be stitched based on at least one template with respect to the first image or at least one template with respect to the second image. Alternatively, the first and second images may be stitched by using respective templates of the first and second images.

Also, a position and size of at least one template according to an embodiment of the present invention may be set in various manners. That is, at least one template according to an embodiment of the present invention may be set differently based on at least one of properties of a target object, a size of a ROI, and a position of a ROI. The properties of the target object according to an embodiment of the present invention may include the age, gender, height, weight, body type, a part to be imaged, and medical history of the target object.

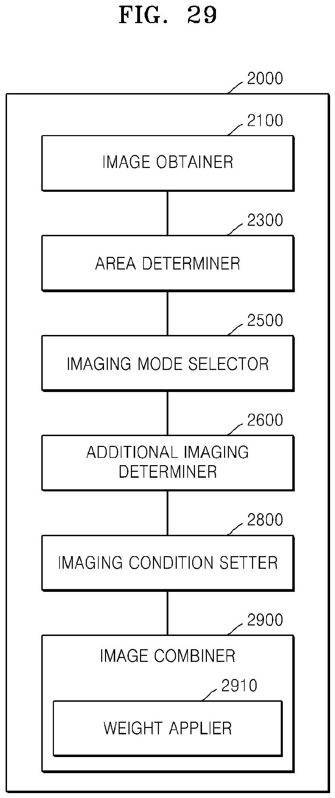

FIG. 29 is a block diagram illustrating an apparatus 2000 that further includes an image combining unit 2900, according to another embodiment of the present invention.

According to an embodiment of the present invention, a second image may include a plurality of templates to be used in combining the second image with a first image.

The image combining unit 2900 may further include a weight applying unit 2910 that applies different weights to a plurality of templates. The image combining unit 2900 may stitch an obtained first image and an obtained second image based on weights applied by using the weight applying unit 2910.

According to an embodiment of the present invention, the image combining unit 2900 may set a plurality of templates T100 through T400 with respect to the first image 240. Also, similarly, the image combining unit 2900 may set a plurality of different templates with respect to the second image 260.

A template in the form of a block that has a fixed size and is set at a fixed position may be vulnerable to a reversion phenomenon between imaged images, particularly, in imaging a target object using an X-ray source of a stepping type, and thus, it may be difficult to obtain an exact X-ray image of a target object. Also, when a template in the form of a block that has a fixed size and is set at a fixed position is used, if the target object moves or image quality of an obtained image of the target object is not uniform, efficiency of image stitching may decrease.

According to the current embodiment of the present invention, at least one of a position and a size of a template may be variably set based on a feature point or a boundary line of a ROI of a target object. For example, at least one of a position and a size of a template may be set such that the template includes a feature point of a ROI of a target object included in the first image 240 (e.g., a feature point P2' of FIG. 12B). That is, at least one of a position and a size of a template may be set such that the template necessarily includes a feature point included in the first image (e.g., a feature point P2' of FIG. 12B).

Also, a template according to an embodiment of the present invention may be set to have a predetermined size at a position nearest to a feature point included in the first image 240 (e.g., a feature point P2' of FIG. 12B), but is not limited thereto.

Also, the template T400 that is set by using the image combining unit 2900 according to the current embodiment of the present invention may include at least one sub-template (e.g., T410 through T450).

According to an embodiment of the present invention, different weights may be applied to a plurality of templates (e.g., T410 through T450) in operation S900 of FIG. 18.

For example, different weights may be respectively applied to sub-templates, for example, such that a weight W1 is applied to a template T410, and a weight W2 is applied to a template T420. For example, weights W1 through W5 applied to a plurality of sub-templates may be different from one another. Also, some of sub-templates may have the same weight, which is different from those of other sub-templates (for example, W1=W4=W5 and W1<W2<W3), but the embodiments of the present invention are not limited thereto.

According to an embodiment of the present invention, by using the plurality of templates T410 through T450 to which weights are applied by using the weight applying unit 2910, the first image 240 and the second image 260 may be stitched. Image stitching is performed based on the applied weights as described above as a data amount to be processed during image stitching may be reduced, and thus, a combination image may be quickly obtained, and a imaging time may be reduced.

For example, elements of an image area (e.g., a pixel value) included in a template to which a relatively high weight (e.g., W3 in the above-described example) is applied may be used frequently in image stitching. On the other hand, elements of an image area included in a template to which a relatively low weight is applied may be considered relatively little in image stitching.

According to an embodiment of the present invention, the image combining unit 2900 may perform an image stitching post-process. For example, the first image 240 and the second image 260 may be stitched smoothly and naturally through a blending operation. Blending between images may include an operation of adjusting a mixing ratio between a first image and a second image near an image stitching portion to express the image stitching portion smoothly and naturally.

Also, a series of processes to appropriately match the first image 240 and the second image 260 may be additionally performed by cropping a portion or the whole of each of the first and second images 240 and 260, rotating the first and second images 240 and 260, or adjusting phases of the first and second images 240 and 260.

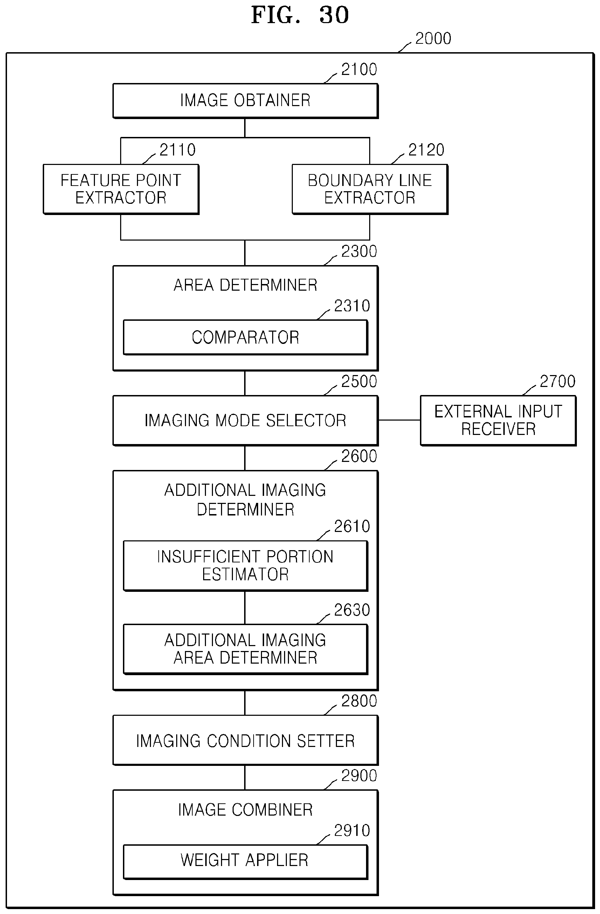

FIG. 30 is a block diagram illustrating an apparatus 2000 for obtaining an X-ray image including a ROI of a target object, according to an embodiment of the present invention.

The apparatus 200 for obtaining an X-ray image including a ROI of a target object may include an image obtaining unit 2100, a feature point extracting unit 2110, a boundary line extracting unit 2120, an area determining unit 2300, a imaging mode providing unit 2400, a imaging mode selecting unit 2500, an additional imaging determining unit 2600, an external input receiving unit 2700, a imaging condition setting unit 2800, and an image combining unit 2900.

The area determining unit 2300 may further include a comparing unit 2310. The additional imaging determining unit 2600 may further include a sufficient portion estimating unit 2610 and an additional imaging area determining unit 2630. The image combining unit 2900 may further include a weight applying unit 2910.

A method of obtaining an image of a ROI of a target object according to an embodiment of the present invention may include: obtaining a first image of the target object; obtaining a second image of the target object; and generating an image including the ROI of the target object by using the first image and the second image. The second image may have a different size from the first image and include a portion or the whole of the ROI. For example, the first image and the second image may have different sizes, and an image including the ROI of the target object may be generated by using the first image and the second image. For example, if a kidney and a portion of a ureter are imaged on the first image, the second image including the other portion of the ureter and a bladder is imaged so that all of the kidney, the ureter, and the bladder are included in a combination image generated by using the first image and the second image. According to the above embodiment, the second image may have a different size from the first image and include only a portion of the ROI. Also, according to the current embodiment of the present invention, the second image including the entire ROI may be obtained.

Also, the second image may be smaller than the first image. In order that an amount of exposure to radiation with respect to the target object does not unnecessarily increase, the second image may be smaller than the first image. In the above embodiment, if a kidney and a portion of a ureter are imaged on the first image, the second image including the other portion of the ureter and a bladder is imaged so that an increase in the amount of exposure to radiation with respect to the target object is prevented and all of the kidney, the ureter, and the bladder are included in a combination image generated by using the first image and the second image.

A method of obtaining an image of a ROI of a target object according to an embodiment of the present invention may include: determining sizes of a first image and a second image that are to be imaged based on a size of the ROI; obtaining the first image of the target object based on the determined size; obtaining the second image of the target object based on the determined size; and generating an image including the ROI of the target object by using the first image and the second image. The second image may have a different size from the first image and include a portion or the whole of the ROI.

For example, when the size of the ROI is the same as or greater than a size of an image that is typically imaged, imaging an image that includes the entire ROI may be dependent upon the skills of the user. Thus, according to an embodiment of the present invention, a method of obtaining an image that includes an ROI regardless of the skills of the user may be provided. For example, sizes of a first image and a second image to be imaged may be preset based on a size of a ROI, and the first image and the second image having the determined sizes may be obtained, and an image including the entire ROI may be generated by using the first and second images. The ROI according to the current embodiment of the present invention may include, for example, a tissue in the target object.

Also, the generating of an image including the ROI of the target object by using the first image and the second image according to an embodiment of the present invention may include generating an image by combining the first image and the second image by overlapping the first image and the second image by a predetermined size. The first image and the second image may be overlapped by a predetermined size to be generated as a single image. A size of an overlapping portion in the first image and the second image is as described above. In addition, the single image may be referred to as a combination image.

Also, an apparatus for obtaining an image of a ROI of a target object according to an embodiment of the present invention may include: an image obtaining unit that is configured to obtain a first image and a second image of the target object and an image generating unit that is configured to generate an image including the ROI of the target object by using the first image and the second image, wherein the second image has a different size from the first image and may include a portion or the whole of the ROI.

Also, the second image may be smaller than the first image.

Also, an apparatus for obtaining an image of a ROI of a target object according to an embodiment of the present invention may include: an image size determining unit that is configured to determine sizes of a first image and a second image to be imaged based on a size of the ROI; an image obtaining unit that is configured to obtain the first image and the second image of the target object based on the determined sizes; and an image generating unit that is configured to generate an image including the ROI of the target object by using the first image and the second image, wherein the second image has a different size from the first image and may include a portion or the whole of the ROI.

Also, the image generating unit may be configured to generate an image by combining the first image and the second image by overlapping the first image and the second image by a predetermined size. The predetermined size by which the first image and the second image are overlapped may be in the range from about 35 mm to about 90 mm.

In the exemplary embodiments described above the images are obtained using X-rays, but the skilled person understands that the disclosed principles are also applicable when the image is obtained using another imaging technology.

According to yet another embodiment of a method of obtaining an image of a region of interest (ROI) of a target object, the method comprises: obtaining a first image of the target object; determining whether the first image includes the whole ROI; determining a missing portion of the ROI when it is determined that the first image does not include the whole ROI; selecting a imaging mode to reimage the missing portion of the ROI, when it is determined that the first image does not include the whole ROI; and obtaining a second image of the target object according to the selected imaging mode, such that the second image includes the missing portion and has a smaller size than the first image. In an embodiment the method may further comprise extracting at least one feature point and/or line related to the ROI from the first image, and the determining whether the first image includes the whole ROI may comprise comparing the at least one feature point and/or line extracted from the first image with at least one feature point and/or line related to the ROI included in a standard image. If the number of feature points in the first image is different from the number of feature points in the standard image, it may be determined that the first image does not include the whole ROI, and/or, if a portion of the at least one line present in the standard image is missing in the first image, it may be determined that the first image does not include the whole ROI. In a possible embodiment the comparing of the at least one feature point and/or line extracted from the first image with the feature point and/or line related to the ROI included in the standard image comprises comparing the number and/or a position of the at least one feature point and/or line related to the ROI included in the standard image and the number and/or a position of the at least one feature point and/or line extracted from the first image. Using feature points and/or lines allows for a fast and effective comparing of the first image with a standard image, and results in a reliable and accurate determining whether the first image includes the whole ROI.

According to the method and apparatus for obtaining an X-ray image including a region of interest (ROI) of a target object, an additional imaging area of a target object which may be obtained when an image of organs that are distributively located in the target object are captured may be appropriately selected to thereby minimize an amount of radiation exposure with respect to the target object. In addition, an unnecessary increase in imaging time due to repeated reimaging may be prevented.

The above-described method may be applied to the apparatus according to embodiments of the present invention. Thus, a description of the apparatus that is similar to the description of the method will not be repeated here.

The above-described embodiments of the present invention may be written as computer programs and may be implemented in general-use digital computers that execute the programs using a computer-readable recording medium.

Examples of the computer-readable recording medium include magnetic storage media (e.g., ROM, floppy disks, hard disks, etc.), optical recording media (e.g., CD-ROMs or DVDs).

The present invention has been described by referring to exemplary embodiments. While the exemplary embodiments have been particularly shown and described, it will be understood by those of ordinary skill in the art that various changes in form and details may be made therein without departing from the spirit and scope of the present invention as defined by the appended claims. Therefore, the exemplary embodiments should be considered in a descriptive sense only and not for purposes of limitation. Therefore, the scope of the present invention is defined not by the detailed description of the exemplary embodiments, but by the appended claims, and all differences within the scope will be construed as being included in the present invention.

**Claims**

1.   A method of obtaining an image of a region of interest (ROI) of a target object, the method comprising:

   obtaining a first image of the target object, wherein a portion of the region of interest is missing in the first image;
   providing a plurality of selectable imaging modes for reimaging the target object; and
   obtaining a second image of the target object according to a selected imaging mode of said plurality of imaging modes, such that the second image includes the missing portion;
   wherein said providing of a plurality of selectable imaging modes is performed such that a selection of an imaging mode can be made after obtaining said first image and before obtaining said second image.

2.   The method of claim 1, wherein the providing of a plurality of imaging modes comprises providing a plurality of imaging modes corresponding with imaging areas having different sizes.

3.   The method of claim 1 or 2, wherein the obtaining of the first image and the obtaining of the second image are performed while the target object remains in a determined position.

4.   The method of any one of the previous claims, wherein the providing of a plurality of imaging modes comprises providing a plurality of imaging modes corresponding with different positions and/or orientations of an image source used to obtain the first and second image.

5.   The method of any one of the previous claims, the method further comprising:

   determining whether the first image includes the whole ROI; and
   selecting an imaging mode of said plurality of selectable imaging modes, if it is determined that the first image does not include the whole ROI;
   wherein the second image imaging mode is selected such that the second image includes a portion or the whole of the ROI.

6.   The method of claim 2 and 5, wherein the determining comprises determining that a portion of the ROI is missing in the first image, and the selecting comprises selecting an imaging mode of said plurality of imaging modes corresponding with an imaging area having the smallest size capable of containing the portion which is missing in the first image.

7.   The method of claim 5 or 6-, wherein the determining whether the first image includes the ROI comprises comparing a standard image of the target object and the first image; wherein preferably the standard image is selected from among previously stored images based on properties of the target object.

8.   The method of claim 7, further comprising extracting at least one feature point and/or line related to the ROI from the first image,
wherein the determining whether the first image includes the ROI comprises comparing the at least one feature point and/or line extracted from the first image with at least one feature point and/or line related to the ROI included in the standard image.

9. The method of any one of the previous claims, wherein the imaging mode includes a whole imaging mode in which the entire ROI of the target object is reimaged and a partial imaging mode in which a portion of the ROI of the target object is reimaged.

10. The method of claim 9 and any one of the claims 5-10, further comprising determining a portion of the ROI to be additionally imaged, when the partial imaging mode is selected,
wherein the second image including the determined portion is obtained.

11. The method of any one of the claims 5-8 or 10, wherein the determining comprises:

estimating a portion of the ROI that is not included in the first image; and
determining an additional imaging area including the estimated portion by using size information of the first image.

12. An apparatus for obtaining an image of a region of interest (ROI) of a target object, the apparatus comprising:

an image obtaining unit configured for obtaining a first image of the target object, wherein a portion of the region of interest is missing in the first image;

an image mode providing unit configured for providing a plurality of selectable imaging modes for reimaging the target object, after having obtained the first image; wherein the imaging obtaining unit is further configured for obtaining a second image according to a selected imaging mode of said plurality of imaging modes, such that the second image includes the missing portion.

13. The apparatus of claim 12, wherein the image mode providing unit is configured for providing a plurality of imaging modes corresponding with imaging areas having different sizes.

14. The apparatus of claim 12 or 13, wherein the image mode providing unit is configured providing a plurality of imaging modes corresponding with different positions and/or orientations of an image source used to obtain the first and second image.

15. The apparatus of any one of the claims 12-14, further comprising:

an area determining unit configured for determining whether the first image includes the whole ROI; and
an imaging mode selecting unit configured for selecting an imaging mode of said plurality of imaging modes to reimage the target object if it is determined that the first image does not include the whole ROI.

**Patentansprüche**

1. Verfahren zur Erzeugung eines Bildes einer Region von Interesse (ROI, Region Of Interest) eines Zielobjekts, wobei das Verfahren umfasst:

Erzeugen eines ersten Bildes des Zielobjekts, wobei ein Abschnitt der Region von Interesse in dem ersten Bild fehlt;
zur Verfügung Stellen einer Mehrzahl von auswählbaren Bildgebungsmodi zur erneuten Bildgebung des Zielobjekts; und
Erzeugen eines zweiten Bildes des Zielobjekts gemäß einem ausgewählten Bildgebungsmodus aus der Mehrzahl von Bildgebungsmodi, so dass das zweite Bild den fehlenden Abschnitt beinhaltet;
wobei das zur Verfügung Stellen einer Mehrzahl von auswählbaren Bildgebungsmodi derart durchgeführt wird, dass eine Auswahl des Bildgebungsmodus nach der Erzeugung des ersten Bildes und vor der Erzeugung des zweiten Bildes getroffen werden kann.

2. Verfahren gemäß Anspruch 1, wobei das zur Verfügung Stellen einer Mehrzahl von Bildgebungsmodi umfasst: zur Verfügung Stellen einer Mehrzahl von Bildgebungsmodi, die Bildgebungsbereichen mit unterschiedlichen Größen entsprechen.

3. Verfahren gemäß Anspruch 1 oder 2, wobei das Erzeugen des ersten Bildes und das Erzeugen des zweiten Bildes durchgeführt werden, während das Zielobjekt in einer bestimmten Position verbleibt.

**4.** Verfahren gemäß einem der vorangegangenen Ansprüche, wobei das zur Verfügung Stellen einer Mehrzahl von Bildgebungsmodi umfasst: zur Verfügung Stellen einer Mehrzahl von Bildgebungsmodi, bei denen die Bildgebungsmodi unterschiedlichen Positionen und/oder Ausrichtungen einer Bildquelle, die zur Erzeugung des ersten und des zweiten Bildes verwendet wird, entsprechen.

**5.** Verfahren gemäß einem der vorangegangenen Ansprüche, wobei das Verfahren weiterhin umfasst:

Feststellen, ob das erste Bild die gesamte ROI beinhaltet; und
Auswählen eines Bildgebungsmodus aus der Mehrzahl von auswählbaren Bildgebungsmodi, wenn festgestellt wird, dass das erste Bild nicht die gesamte ROI beinhaltet;
wobei der zweite Bildgebungsmodus derart ausgewählt wird, dass das zweite Bild die gesamte ROI oder einen Abschnitt davon beinhaltet.

**6.** Verfahren gemäß den Ansprüchen 2 und 5, wobei das Feststellen die Feststellung umfasst, dass ein Abschnitt der ROI in dem ersten Bild fehlt, und das Auswählen umfasst, dass ein Bildgebungsmodus aus der Mehrzahl von Bildgebungsmodi ausgewählt wird, der einem Bildgebungsbereich mit der kleinstmöglichen Größe, die in der Lage ist, den in dem ersten Bild fehlenden Abschnitt zu beinhalten, entspricht.

**7.** Verfahren gemäß Anspruch 5 oder 6, wobei das Feststellen, ob das erste Bild die ROI beinhaltet, umfasst: Vergleichen eines Standardbildes des Zielobjekts mit dem ersten Bild;
wobei das Standardbild vorzugsweise aus zuvor gespeicherten Bildern basierend auf Eigenschaften des Zielobjekts ausgewählt wird.

**8.** Verfahren gemäß Anspruch 7, das weiterhin umfasst: Extrahieren wenigstens eines Merkmalspunktes und/oder wenigstens einer Merkmalslinie in Verbindung mit der ROI aus dem ersten Bild,
wobei das Feststellen, ob das erste Bild die ROI beinhaltet, umfasst: Vergleichen des wenigstens einen Merkmalspunktes und/oder der wenigstens einen Merkmalslinie aus dem ersten Bild mit wenigstens einem Merkmalspunkt und/oder wenigstens einer Merkmalslinie in Verbindung mit der in dem Standardbild enthaltenen ROI.

**9.** Verfahren gemäß einem der vorangegangenen Ansprüche, wobei der Bildgebungsmodus beinhaltet: einen Gesamtbildgebungsmodus, in dem die gesamte ROI des Zielobjektes einer erneuten Bildgebung unterzogen wird, und einen Teilbildgebungsmodus, in dem ein Abschnitt der ROI des Zielobjektes einer erneuten Bildgebung unterzogen wird.

**10.** Verfahren gemäß Anspruch 9 und gemäß einem der Ansprüche 5 bis 10, das weiterhin umfasst: Feststellen eines Abschnitts der ROI, der zusätzlich einer Bildgebung unterzogen werden soll, wenn der Teilbildgebungsmodus ausgewählt wird,
wobei das zweite Bild, das den festgelegten Abschnitt beinhaltet, erzeugt wird.

**11.** Verfahren gemäß einem der Ansprüche 5 bis 8 oder Anspruch 10, wobei das Feststellen umfasst:

Schätzen eines Abschnitts der ROI, der nicht in dem ersten Bild beinhaltet ist; und
Feststellen eines zusätzlichen Bildgebungsbereichs, der den geschätzten Abschnitt beinhaltet, mit Hilfe von Größeninformationen aus dem ersten Bild.

**12.** Vorrichtung zur Erzeugung eines Bildes einer Region von Interesse (ROI, Region Of Interest) eines Zielobjekts, wobei die Vorrichtung umfasst:

eine Bilderzeugungseinheit, die konfiguriert ist zum Erzeugen eines ersten Bildes des Zielobjekts, wobei ein Abschnitt der Region von Interesse in dem ersten Bild fehlt;
eine Einheit zum zur Verfügung Stellen von Bildgebungsmodi, die konfiguriert ist zum zur Verfügung Stellen einer Mehrzahl von auswählbaren Bildgebungsmodi zur erneuten Bildgebung des Zielobjekts, nachdem das erste Bild erzeugt worden ist; wobei die Bilderzeugungseinheit weiterhin konfiguriert ist zum Erzeugen eines zweiten Bildes gemäß einem ausgewählten Bildgebungsmodus aus der Mehrzahl von Bildgebungsmodi, so dass das zweite Bild den fehlenden Abschnitt beinhaltet.

**13.** Vorrichtung gemäß Anspruch 12, wobei die Einheit zum zur Verfügung Stellen von Bildgebungsmodi konfiguriert ist zum zur Verfügung Stellen einer Mehrzahl von Bildgebungsmodi, die Bildgebungsbereichen mit unterschiedlichen Größen entsprechen.

**14.** Vorrichtung gemäß Anspruch 12 oder 13, wobei die Einheit zum zur Verfügung Stellen von Bildgebungsmodi konfiguriert ist zum zur Verfügung Stellen einer Mehrzahl von Bildgebungsmodi, bei denen die Bildgebungsmodi unterschiedlichen Positionen und/oder Ausrichtungen einer Bildquelle, die zur Erzeugung des ersten und des zweiten Bildes verwendet wird, entsprechen.

**15.** Vorrichtung gemäß einem der Ansprüche 12 bis 14, die weiterhin umfasst:

eine Bereichsfeststellungseinheit, die konfiguriert ist zum Feststellen, ob das erste Bild die gesamte ROI beinhaltet; und

eine Bildgebungsmodusauswahleinheit, die konfiguriert ist zum Auswählen eines Bildgebungsmodus aus der Mehrzahl von Bildgebungsmodi, um das Zielobjekt einer erneuten Bildgebung zu unterziehen, wenn festgestellt wird, dass das erste Bild nicht die gesamte ROI beinhaltet.

**Revendications**

**1.** Procédé permettant d'obtenir une image d'une région d'intérêt (ROI, region of interest) d'un objet cible, le procédé comprenant :

l'obtention d'une première image de l'objet cible, une partie de la région d'intérêt étant manquante dans la première image,

la mise à disposition d'une pluralité de modes d'imagerie sélectionnables visant à soumettre l'objet cible à un nouveau processus d'imagerie, et

l'obtention d'une deuxième image de l'objet cible selon un mode d'imagerie sélectionné parmi ladite pluralité de modes d'imagerie, de manière que la deuxième image comporte la partie manquante ;

ladite mise à disposition d'une pluralité de modes d'imagerie sélectionnables s'effectuant de manière que la sélection du mode d'imagerie puisse être réalisée après l'obtention de ladite première image et avant l'obtention de ladite deuxième image.

**2.** Procédé selon la revendication 1, dans lequel la mise à disposition d'une pluralité de modes d'imagerie comprend la mise à disposition d'une pluralité de modes d'imagerie en correspondance avec des zones d'imagerie de tailles différentes.

**3.** Procédé selon la revendication 1 ou 2, dans lequel l'obtention de la première image et l'obtention de la deuxième image s'effectuent pendant que l'objet cible reste dans une position définie.

**4.** Procédé selon l'une quelconque des revendications précédentes, dans lequel la mise à disposition d'une pluralité de modes d'imagerie comprend la mise à disposition d'une pluralité de modes d'imagerie en correspondance avec différentes positions et/ou orientations d'une source d'image servant à l'obtention de la première et de la deuxième image.

**5.** Procédé selon l'une quelconque des revendications précédentes, le procédé comprenant en outre :

la détermination du fait que la première image comporte la totalité de la ROI ou non, et

la sélection d'un mode d'imagerie parmi ladite pluralité de modes d'imagerie sélectionnables, s'il a été déterminé que la première image ne comprend pas la totalité de la ROI ;

le mode d'imagerie de la deuxième image étant sélectionné de manière que la deuxième image comporte une partie ou la totalité de la ROI.

**6.** Procédé selon les revendication 2 et 5, dans lequel la détermination comprend la détermination du fait qu'une partie de la ROI étant manquante dans la première image, et la sélection comprend la sélection d'un mode d'imagerie parmi ladite pluralité de modes d'imagerie en correspondance avec une zone d'imagerie présentant la plus petite taille apte à contenir la partie qui est manquante dans la première image.

**7.** Procédé selon la revendication 5 ou 6, dans lequel la détermination du fait que la première image comporte la ROI ou non comprend la comparaison d'une image standard de l'objet cible et de la première image ;

l'image standard étant de préférence sélectionnée parmi des images précédemment stockées en fonction des propriétés de l'objet cible.

8. Procédé selon la revendication 7, comprenant en outre l'extraction, hors de la première image, d'au moins un point et/ou une ligne caractéristique en lien avec la ROI ;
la détermination du fait que la première image comporte la ROI ou non comprenant la comparaison de l'au moins un point et/ou une ligne caractéristique extrait(e) de la première image et d'au moins un point et/ou une ligne caractéristique en lien avec la ROI incluse dans l'image standard.

9. Procédé selon l'une quelconque des revendications précédentes, dans lequel le mode d'imagerie comporte un mode d'imagerie totale dans lequel la totalité de la ROI de l'objet cible subit un nouveau processus d'imagerie, et un mode d'imagerie partielle dans lequel une partie de la ROI de l'objet cible subit un nouveau processus d'imagerie.

10. Procédé selon la revendication 9 et l'une quelconque des revendications 5 à 10, comprenant en outre la détermination d'une partie de la ROI devant subir en outre un processus d'imagerie lorsque le mode d'imagerie partielle a été sélectionné;
la deuxième image comportant la partie déterminée étant ainsi obtenue.

11. Procédé selon l'une quelconque des revendications 5 à 8 ou 10, dans lequel la détermination comprend :

l'estimation d'une partie de la ROI qui n'est pas incluse dans la première image, et
la détermination d'une zone d'imagerie supplémentaire comportant la partie estimée, grâce à l'exploitation d'informations de taille de la première image.

12. Appareil permettant d'obtenir une image d'une région d'intérêt (ROI, region of interest) d'un objet cible, l'appareil comprenant :

une unité d'obtention d'image conçue pour obtenir une première image de l'objet cible, une partie de la région d'intérêt étant manquante dans la première image,
une unité de mise à disposition de mode d'imagerie conçue pour mettre à disposition une pluralité de modes d'imagerie sélectionnables visant à soumettre l'objet cible à un nouveau processus d'imagerie, après obtention de la première image ; ladite unité d'obtention d'image étant en outre conçue pour obtenir une deuxième image selon un mode d'imagerie sélectionné parmi ladite pluralité de modes d'imagerie, de manière que la deuxième image comporte la partie manquante.

13. Appareil selon la revendication 12, dans lequel l'unité de mise à disposition de mode d'imagerie est conçue pour mettre à disposition une pluralité de modes d'imagerie en correspondance avec des zones d'imagerie de tailles différentes.

14. Appareil selon la revendication 12 ou 13, dans lequel l'unité de mise à disposition de mode d'imagerie est conçue pour mettre à disposition une pluralité de modes d'imagerie en correspondance avec différentes positions et/ou orientations d'une source d'image servant à l'obtention de la première et de la deuxième image.

15. Appareil selon l'une quelconque des revendications 12 à 14, comprenant en outre :

une unité de détermination de zones conçue pour déterminer si la première image comporte la totalité de la ROI ou non, et
une unité de sélection de mode d'imagerie conçue pour sélectionner un mode d'imagerie parmi ladite pluralité de modes d'imagerie afin de soumettre l'objet cible à un nouveau processus d'imagerie s'il est déterminé que la première image ne comprend pas la totalité de la ROI.

FIG. 1

# FIG. 2A

MOVED

# FIG. 2B

# FIG. 2C

270

250

# FIG. 3A

START

DETERMINE ROI TO BE IMAGED — S10

DIVIDE ROI INTO PLURALITY OF IMAGING REGIONS — S12

OBTAIN INDIVIDUAL IMAGES
CORRESPONDING TO PLURALITY OF IMAGING REGIONS — S20

OBTAIN ENTIRE IMAGE — S22

END

# FIG. 3B

START

↓

OBTAIN FIRST IMAGE OF OBJECT — S100

↓

DETERMINE WHETHER FIRST IMAGE INCLUDES ROI — S300

↓

SELECT IMAGING MODE TO RE-IMAGE OBJECT — S500

↓

OBTAIN SECOND IMAGE OF OBJECT
ACCORDING TO SELECTED IMAGING MODE — S700

↓

END

# FIG. 4

S300

DETERMINE WHETHER
FIRST IMAGE INCLUDES ROI

S310

COMPARE REFERENCE IMAGE
WITH FIRST IMAGE

FIG. 5A

FIG. 5B

FIG. 5C

FIG. 5D

FIG. 5E

# FIG. 6

```
                    ┌─────────┐
                    │  START  │
                    └─────────┘
                         │
                         ▼
    ┌────────────────────────────────────────────┐
    │        OBTAIN FIRST IMAGE OF OBJECT         │──── S100
    └────────────────────────────────────────────┘
                         │
                         ▼
    ┌────────────────────────────────────────────┐
    │   EXTRACT AT LEAST ONE FEATURE POINT FROM   │──── S200
    │                FIRST IMAGE                  │
    └────────────────────────────────────────────┘
                         │
                         ▼
    ┌────────────────────────────────────────────┐
    │   DETERMINE WHETHER FIRST IMAGE INCLUDES ROI│
    │                              ┌─S311         │
    │   ┌──────────────────────────────────────┐ │──── S300
    │   │ COMPARE FEATURE POINT OF FIRST IMAGE  │ │
    │   │      WITH FEATURE POINT OF            │ │
    │   │         REFERENCE IMAGE              │ │
    │   └──────────────────────────────────────┘ │
    └────────────────────────────────────────────┘
                         │
                         ▼
    ┌────────────────────────────────────────────┐
    │        SELECT MODE TO RE-IMAGE OBJECT       │──── S500
    └────────────────────────────────────────────┘
                         │
                         ▼
    ┌────────────────────────────────────────────┐
    │       OBTAIN SECOND IMAGE OF OBJECT         │──── S700
    │   ACCORDING TO SELECTED IMAGING MODE        │
    └────────────────────────────────────────────┘
                         │
                         ▼
                    ┌─────────┐
                    │   END   │
                    └─────────┘
```

FIG. 7C

FIG. 7B

FIG. 7A

FIG. 7E

FIG. 7D

P1 P2 P3 P4 P5

# FIG. 8

START

OBTAIN FIRST IMAGE OF OBJECT — S100

EXTRACT FIRST BOUNDARY LINE OF ROI FROM FIRST IMAGE — S210

DETERMINE WHETHER FIRST IMAGE INCLUDES ROI — S300

S313

COMPARE FIRST BOUNDARY LINE OF ROI OF FIRST IMAGE
WITH SECOND BOUNDARY LINE OF ROI OF REFERENCE IMAGE

SELECT IMAGING MODE TO RE-IMAGE TARGET OBJECT — S500

OBTAIN SECOND IMAGE OF OBJECT ACCORDING TO
SELECTED IMAGING MODE — S700

END

FIG. 9A  FIG. 9B  FIG. 9C  FIG. 9D  FIG. 9E

## FIG. 10A

```
                    START
                      │
                      ▼
         OBTAIN FIRST IMAGE OF OBJECT ──── S100
                      │
                      ▼
              DETERMINE WHETHER ──── S300 ──YES──→ END IMAGE
           FIRST IMAGE INCLUDES ROI                 CAPTURING
                      │
                     NO
                      │
                      ▼
              SELECT IMAGING MODE ──── S500
                  │            │
          ┌───────┘            └───────┐
          ▼                            ▼
  SELECT FIRST IMAGING MODE ─S502   SELECT PARTIAL IMAGING MODE ─S504
          │                            │
          ▼                            ▼
     SELECT IMAGING ─S610         SELECT IMAGING ─S630
  CONDITION ACCORDING TO       CONDITION ACCORDING TO
    FIRST IMAGING MODE          PARTIAL IMAGING MODE
          │                            │
          └──────────┬─────────────────┘
                     ▼
          OBTAIN SECOND IMAGE OF ──── S700
              OBJECT ACCORDING TO
          SELECTED IMAGING CONDITION
                     │
                     ▼
              IS PORTION ──── S702
         LACKING IN ROI INCLUDED IN ──NO──→ (back to SELECT IMAGING MODE)
            SECOND IMAGE?
                     │
                    YES
                     ▼
                    END
```

## FIG. 10B

EP 2 813 183 B1

# FIG. 11

```
┌─────────────────────────────────────────────────┐
│         SELECT PARTIAL IMAGING MODE              │─── S504
└─────────────────────────────────────────────────┘
                        │
                        ▼
┌─────────────────────────────────────────────────┐
│    DETERMINE PORTION TO BE ADDITIONALLY IMAGED   │
│                                        ⟋ S511    │
│   ┌─────────────────────────────────────────┐    │
│   │ ESTIMATE PORTION OF ROI NOT INCLUDED IN  │    │
│   │            FIRST IMAGE                    │    │─── S510
│   └─────────────────────────────────────────┘    │
│                                        ⟋ S513    │
│   ┌─────────────────────────────────────────┐    │
│   │ DETERMINE AREA THAT INCLUDES ESTIMATED   │    │
│   │            PORTION                        │    │
│   └─────────────────────────────────────────┘    │
└─────────────────────────────────────────────────┘
                        │
                        ▼
┌─────────────────────────────────────────────────┐
│     SELECT IMAGING CONDITION ACCORDING TO        │─── S630
│          PARTIAL IMAGING MODE                    │
└─────────────────────────────────────────────────┘
                        │
                        ▼
┌─────────────────────────────────────────────────┐
│    OBTAIN SECOND IMAGE OF OBJECT ACCORDING TO    │─── S700
│        SELECTED IMAGING CONDITION                │
└─────────────────────────────────────────────────┘
                        │
                        ▼
                  ╭───────────╮
                  │    END    │
                  ╰───────────╯
```

## FIG. 12A

REFERENCE IMAGE 200

## FIG. 12B

FIRST IMAGE 240

## FIG. 13A

REFERENCE IMAGE 200

## FIG. 13B

FIRST IMAGE 240

# FIG. 14

```
                    ┌─────────┐
                    │  START  │
                    └─────────┘
                         │
                         ▼
┌──────────────────────────────────────────┐
│      RECEIVE ADDITIONAL IMAGING AREA AS   │
│          EXTERNAL INPUT SIGNAL            │── S520
└──────────────────────────────────────────┘
                         │
                         ▼
┌──────────────────────────────────────────┐
│    SELECT IMAGING CONDITION ACCORDING TO  │
│          PARTIAL IMAGING MODE             │── S630
└──────────────────────────────────────────┘
                         │
                         ▼
┌──────────────────────────────────────────┐
│    OBTAIN SECOND IMAGE OF OBJECT ACCORDING │
│       TO SELECTED IMAGING CONDITION       │── S700
└──────────────────────────────────────────┘
                         │
                         ▼
                    ┌─────────┐
                    │   END   │
                    └─────────┘
```

# FIG. 15A

EP 2 813 183 B1

# FIG. 15B

240

244

245

Worklist ▸ Acquisition ▸ Review

| Procedure | Protocol |
|-----------|----------|
| 📂 Undefined study | ▱ ABDOMEN ERECT |

| < 1/1 > | < 1/1 > |

Retake  Q/A

Confirm  Send

Suspend   End Exam

EP 2 813 183 B1

# FIG. 15C

EP 2 813 183 B1

# FIG. 16

# FIG. 17

# FIG. 18

START

DETERMINE ADDITIONAL IMAGING AREA — S510

SELECT IMAGING CONDITION ACCORDING TO PARTIAL IMAGING MODE — S630

OBTAIN SECOND IMAGE OF OBJECT ACCORDING TO SELECTED IMAGING CONDITION — S700

COMBINE FIRST IMAGE AND SECOND IMAGE — S900

END

# FIG. 19

FIRST IMAGE 240    SECOND IMAGE    COMBINATION IMAGE 270

+ = 

260

# FIG. 20A

240

T100

# FIG. 20B

240

T100

260

FIG. 21A

FIG. 21B

FIG. 22

FIG. 23

## FIG. 24

# FIG. 25

2000

2100

IMAGE OBTAINER

2300

AREA DETERMINER

2500

IMAGING MODE
SELECTOR

2600

ADDITIONAL IMAGING
DETERMINER

2610

INSUFFICIENT PORTION
ESTIMATOR

2630

ADDITIONAL
IMAGING AREA
DETERMINER

# FIG. 26

```
                                    2000
  ┌─────────────────────────────────────────────────────┐
  │                              2100                     │
  │        ┌──────────────────────────────┐              │
  │        │       IMAGE OBTAINER         │              │
  │        └──────────────────────────────┘              │
  │                              2300                     │
  │        ┌──────────────────────────────┐              │
  │        │       AREA DETERMINER        │              │
  │        └──────────────────────────────┘              │
  │                              2500           2700      │
  │        ┌──────────────────────────────┐  ┌────────┐  │
  │        │   IMAGING MODE SELECTOR      │──│EXTERNAL│  │
  │        └──────────────────────────────┘  │ INPUT  │  │
  │                                          │RECEIVER│  │
  │                              2600        └────────┘  │
  │        ┌──────────────────────────────┐              │
  │        │ ADDITIONAL IMAGING           │              │
  │        │ DETERMINER                   │              │
  │        │              2610            │              │
  │        │  ┌────────────────────────┐  │              │
  │        │  │ INSUFFICIENT PORTION   │  │              │
  │        │  │ ESTIMATOR              │  │              │
  │        │  └────────────────────────┘  │              │
  │        │              2630            │              │
  │        │  ┌────────────────────────┐  │              │
  │        │  │ ADDITIONAL             │  │              │
  │        │  │ IMAGING AREA           │  │              │
  │        │  │ DETERMINER             │  │              │
  │        │  └────────────────────────┘  │              │
  │        └──────────────────────────────┘              │
  └─────────────────────────────────────────────────────┘
```

FIG. 27

```
                                           2000
┌─────────────────────────────────────────────┐
│                                     2100      │
│   ┌─────────────────────────────────┐         │
│   │        IMAGE OBTAINER           │         │
│   └─────────────────────────────────┘         │
│                                     2300      │
│   ┌─────────────────────────────────┐         │
│   │        AREA DETERMINER          │         │
│   └─────────────────────────────────┘         │
│                                     2500      │
│   ┌─────────────────────────────────┐         │
│   │      IMAGING MODE SELECTOR      │         │
│   └─────────────────────────────────┘         │
│                                     2600      │
│   ┌─────────────────────────────────┐         │
│   │      ADDITIONAL IMAGING         │         │
│   │         DETERMINER              │         │
│   └─────────────────────────────────┘         │
│                                     2800      │
│   ┌─────────────────────────────────┐         │
│   │    IMAGING CONDITION SETTER     │         │
│   └─────────────────────────────────┘         │
└─────────────────────────────────────────────┘
```

# FIG. 28

2000

2100
IMAGE OBTAINER

2300
AREA DETERMINER

2500
IMAGING MODE SELECTOR

2600
ADDITIONAL IMAGING DETERMINER

2800
IMAGING CONDITION SETTER

2900
IMAGE COMBINER

# FIG. 29

IMAGE OBTAINER — 2100

AREA DETERMINER — 2300

IMAGING MODE SELECTOR — 2500

ADDITIONAL IMAGING DETERMINER — 2600

IMAGING CONDITION SETTER — 2800

IMAGE COMBINER — 2900

WEIGHT APPLIER — 2910

2000

## FIG. 30

2000

2100
IMAGE OBTAINER

2110
FEATURE POINT EXTRACTOR

2120
BOUNDARY LINE EXTRACTOR

2300
AREA DETERMINER

2310
COMPARATOR

2500
IMAGING MODE SELECTOR

2700
EXTERNAL INPUT RECEIVER

2600
ADDITIONAL IMAGING DETERMINER

2610
INSUFFICIENT PORTION ESTIMATOR

2630
ADDITIONAL IMAGING AREA DETERMINER

2800
IMAGING CONDITION SETTER

2900
IMAGE COMBINER

2910
WEIGHT APPLIER

# FIG. 31

FIG. 32

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007260027 A
- US 7114849 B
- US 20100091949 A
- US 2006241370 A
- US 2008037714 A
- US 2007071172 A
- US 2006198499 A